# EUROPEAN PATENT APPLICATION

(11) **EP 4 458 955 A1**
(43) Date of publication of application: **06.11.2024**
(21) Application number: 22916800.0
(22) Date of filing: 29.12.2022
(51) Int. Cl.: C12N 5/0793, A61K 48/00, A61P 25/00

(54) **FACTOR FOR DIRECT CONVERSION OF MOTOR NERVE CELLS**

(30) Priority: 29.12.2021 KR 20210190556
(71) Applicant: Standup Therapeutics Inc., Seoul 04418 (KR); Yoo, Junsang, Seoul 04418 (KR); Shim, Hyun Soo, Seoul 04572 (KR)
(72) Inventor: CHANG, Yujung, Seoul 04616 (KR); YOO, Junsang, Seoul 04418 (KR); SHIM, Hyun Soo, Seoul 04572 (KR)
(74) Representative: Vossius & Partner Patentanwälte Rechtsanwälte mbB
(86) International application number: PCT/KR2022/021615
(87) International publication number: WO 2023/128659

(57) **Abstract**

The present application relates to a novel differentiation factor for a direct cell-conversion of a somatic cell to a motor neuron and to a use thereof.

## Description

### Technical Field

The present application relates to a novel converting factor for direct cell-conversion into a motor neuron.

The present application relates to a composition including the novel converting factor.

The present application relates to a direct cell-conversion method using the composition.

The present application relates to various uses of the novel converting factor.

### Background Art

Many stem cells are used in regenerative medicine. Stem cells are pluripotent cells that can be converted into all types of cells that make up the human body. Theoretically, the stem cells can be converted into all kinds of functional cells and can proliferate indefinitely. Stem cells include embryonic stem cells, adult stem cells, spermatogonial stem cells, and dedifferentiated stem cells (also referred to as induced pluripotent stem cells) according to their origins.

However, depending on the type, stem cells have problems of ethical issues, stability, and low differentiation efficiency. In addition, dedifferentiated stem cells have problems of teratoma formation and low differentiation efficiency.

Since stem cells have such various problems, new technologies are required in the field of regenerative medicine.

Direct cell-conversion has recently been popular. Direct cell-conversion has a difference in that it directly induces conversion into the desired type of cells without involving a process of reprogramming cells into induced pluripotent stem cells and redifferentiating them into the desired type of cells.

That is, it is a method of inducing conversion between completely different types of mature (differentiated) cells. However, since the conventional direct cell-conversion technique has the limitation of low efficiency, a new method capable of overcoming the limitation is required.

### Disclosure

### Technical Problem

An objective of the present application is to provide a novel converting factor for direct cell-conversion into motor neurons. In particular, the novel converting factor relates to a Glutathione S-transferase A4 (Gsta4) protein or a nucleic acid encoding the Gsta4 protein.

Another objective of the present application is to provide a composition containing the novel converting factor.

A further objective of the present application is to provide a direct cell-conversion method using the composition.

A yet further objective of the present application is to provide various uses of the novel converting factor.

### Technical Solution

This application is intended to solve the above-mentioned problems,

As an embodiment, the present application provides a composition for a direct cell-conversion, comprising:
a Gsta4(Glutathione S-Transferase Alpha 4) protein or a nucleic acid encoding the same.
The composition may further comprise ii) at least one protein selected from Ascl1, Brn2, Myt1L, Hb9, ISL1, Lhx3, Ngn2 and NeuroDl; or a nucleic acid encoding the selected at least one protein.
The composition is characterized in that Hb9 and Lhx3 are selected in ii).
Wherein, the Ascl1 protein may be a SEQ ID NO: 17, and the nucleic acid encoding the Ascl1 protein may have the sequence of SEQ ID NO: 18;
The Brn2 protein may be a SEQ ID NO: 19, and the nucleic acid encoding the Brn2 protein may have the sequence of SEQ ID NO: 20;
The MytlL protein may be a SEQ ID NO: 21, and the nucleic acid encoding the MytlL protein may have the sequence of SEQ ID NO: 22;
The Hb9 protein may be a SEQ ID NO: 13, and the nucleic acid encoding the Hb9 protein may have the sequence of SEQ ID NO: 14;
The Isl1 protein may be a SEQ ID NO: 23, and the nucleic acid encoding the Isl1 protein may have the sequence of SEQ ID NO: 24;
The Lhx3 protein may be a SEQ ID NO: 15, and the nucleic acid encoding the Lhx3 protein may have the sequence of SEQ ID NO: 16;
The Ngn2 protein may be a SEQ ID NO: 25, and the nucleic acid encoding the Ngn2 protein can have the sequence of SEQ ID NO: 26;
The NeuroDl protein may be a SEQ ID NO: 27, and the nucleic acid encoding the NeuroDl protein may have the sequence of SEQ ID NO: 28.

As another embodiment, the present application may provide a vector for expressing a converting factor for a direct cell-conversion, wherein the direct cell-conversion is that a somatic cell is directly converted into an induced motor neuron, and the vector comprising:
i) a nucleic acid encoding Gsta4 protein; and ii) a promoter,

Wherein, the ii) is operably linked to the i).

The vector may further comprise iii) a nucleic acid encoding at least one protein selected from Ascl1, Brn2, Myt1L, Hb9, Isll, Lhx3, Ngn2 and NeuroDl.

the present application may provide a vector for expressing a converting factor for a direct cell-conversion, wherein the direct cell-conversion is that a somatic cell is directly converted into an induced motor neuron, and the vector may comprise at least a first vector and a second vector,

the first vector comprising:
i) a nucleic acid encoding Gsta4 protein; and ii) a promoter,
wherein the ii) is operably linked to the i),
the second vector comprising:
   iii) a nucleic acid encoding at least one protein selected from Ascl1, Brn2, Myt1L, Hb9, Isll, Lhx3, Ngn2 and NeuroDl; and iv) a promoter,
   wherein the iv) is operably linked to the iii),
   wherein the ii) and the iv) are same or different,
   wherein the iii) may be included in one vector or in two or more vectors.

The vector may be a viral vector or a non-viral vector, wherein the viral vector may be one or more selected from a retrovirus, a lentivirus, an adenovirus, an adeno-associated virus (AAV), a vaccinia virus, a poxvirus, a human immunodeficiency virus (HIV), a murine leukemia virus (MLV), an Avian sarcoma/leukosis (ASLV), a Spleen necrosis virus (SNV), a Rous sarcoma virus (RSV), a Mouse mammary tumor virus (MMTV), a Herpes simplex virus, an episomal, and a herpes simplex virus.

As another embodiment, the present application may provide a method for directly converting a somatic cell into an induced motor neuron, comprising: introducing a composition comprising a Gsta4 protein or a nucleic acid encoding the Gsta4 protein into a somatic cell, wherein iPSCs (induced pluripotent stem cells) are not generated by the method.

the somatic cell may be one or more selected from a fibroblast, an epithelial cell, an endothelial cell, a muscle cell, a nerve cell, a hair cell, a hair root cell, a hair follicle cell, an oral epithelial cell, a somatic cell extracted from urine, a gastric mucosal cell, a goblet cell, a gastrin cell/G cell, a B cell, a pericyte cell, an astrocyte, a blood cell and an oligodendrocyte progenitor cell.

Via the introduction, the somatic cell may be converted into an induced motor neuron within 1 to 7 weeks.

As another embodiment, the present application may provide a pharmaceutical composition for preventing or treating a nervous system disease comprising a Gsta4 protein for a direct cell-conversion of a somatic cell into an induced motor neuron.

In addition, the present application may provide a pharmaceutical composition for preventing or treating a nervous system disease comprising a somatic cell introduced with a nucleic acid encoding Gsta4 protein for a direct cell-conversion of a somatic cell into an induced motor neuron.

The pharmaceutical composition may further comprise a nucleic acid encoding at least one protein selected from Ascl1, Brn2, Myt1L, Hb9, Isll, Lhx3, Ngn2 and NeuroDl.

the present application may provide a pharmaceutical composition for preventing or treating a nervous system disease comprising a motor neuron overexpressing direct cell-conversion factors for a direct cell-conversion of a somatic cell into an induced motor neuron, wherein the motor neuron may overexpress one or more direct cell-conversion factor selected from Gsta4, Ascl1 (Achaete-scute homolog 1), Brn2 (POU Class 3 Homeobox 2), MytlL (Myelin Transcription Factor 1 Like), Hb9 (Homeobox HB9), ISL1 (ISL LIM homeobox 1), Lhx3 (LIM homeobox 3), Ngn2 (neurogenin 2) and NeuroDl (neuronal differentiation 1).

As another embodiment, the present application may provide a method for preventing or treating a nervous system disease, the method comprising:
administering i) or ii) below to a subject: i) a Gsta4(Glutathione S-Transferase Alpha 4) protein; or ii) a Gsta4(Glutathione S-Transferase Alpha 4) protein; and at least one protein selected from Ascl1, Brn2, Myt1L, Hb9, Isll, Lhx3, Ngn2 and NeuroDl;
wherein the method does not generate iPSCs (induced pluripotent stem cells).

The present application may provide a method for preventing or treating a nervous system disease, the method comprising: administering a somatic cell introduced with a Gsta4 protein or a nucleic acid encoding the Gsta4 protein to a subject.

The present application may provide a method for preventing or treating a nervous system disease, the method comprising; administering to a subject an induced motor neuron by introducing a composition comprising a Gsta4 protein or a nucleic acid encoding the Gsta4 protein into a somatic cell.

The nervous system disease may be one or more selected from spinal cord injury, Parkinson's disease, stroke, amyotrophic spinal lateral sclerosis, motor nerve damage, traumatic peripheral nerve damage, ischemic brain damage, neonatal hypoxic-ischemic brain damage, cerebral palsy, epilepsy, intractable epilepsy, Alzheimer's disease, congenital metabolic nervous system disease, traumatic brain injury, motor nerve cell damage or a disease caused by the motor nerve cell damage.

### Advantageous Effects

According to this application, the effects described below can be obtained.

First, according to the present application, it is possible to provide a novel converting factor for direct cell-conversion into motor neurons. Since the novel converting factor enables effective differentiation of somatic cells into motor neurons, the novel converting factor may be useful in the treatment of nerve system diseases.

### Description of Drawings

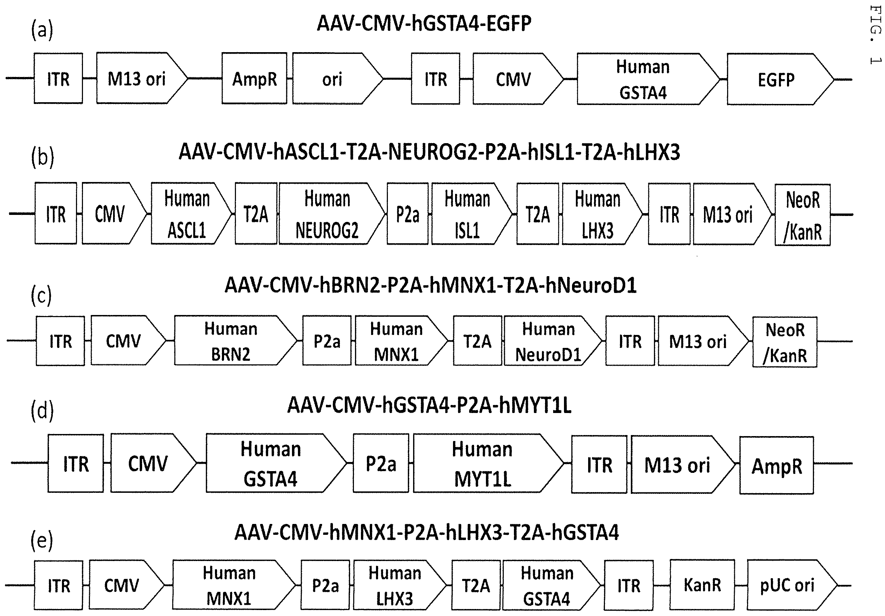

FIG. 1 is a schematic diagram of AAV vectors used in the embodiment of the present invention.
(a) is a schematic diagram of an AAV vector containing Gsta4; (b) is a schematic diagram of an AAV vector including Ascl1, Isll, Lhx3, and Ngn2 (also called Neurog2); (c) is a schematic diagram of an AAV vector containing Brn2, Hb9 (also called Mnx1), and NeuroDl; (d) is a schematic diagram of an AAV vector containing Gsta4 and Myt1l; and (e) is a schematic diagram of an AAV vectors containing Gsta4, Hb9, and Lhx3.

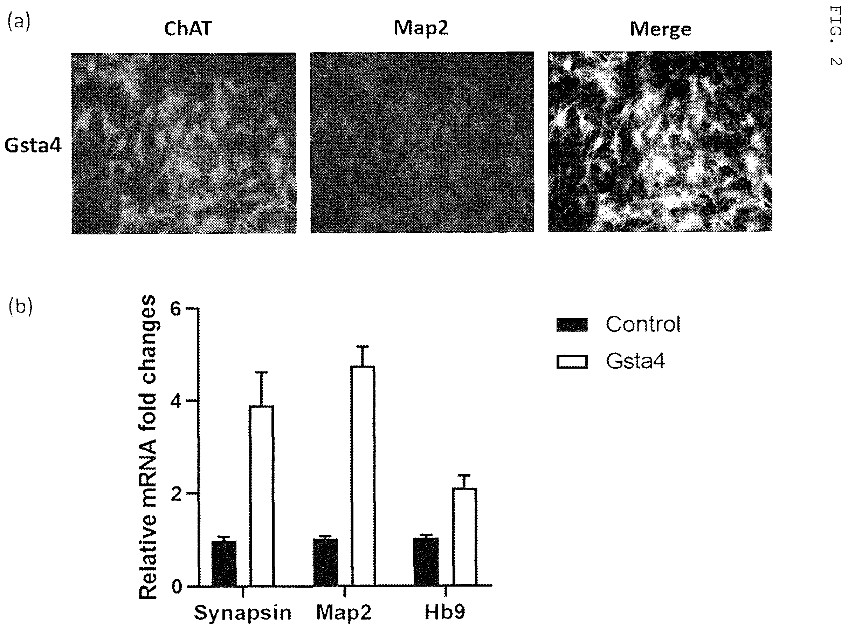

FIG. 2 shows a result of confirming the expression of neuronal markers (ChaT and Map2) to determine the degree of direct cell-conversion into motor neurons when a novel converting factor, Gsta4, is introduced into mouse-derived fibroblasts, in which (a) is the result of immunofluorescence staining, and (b) is the numerical value of the result of (a).

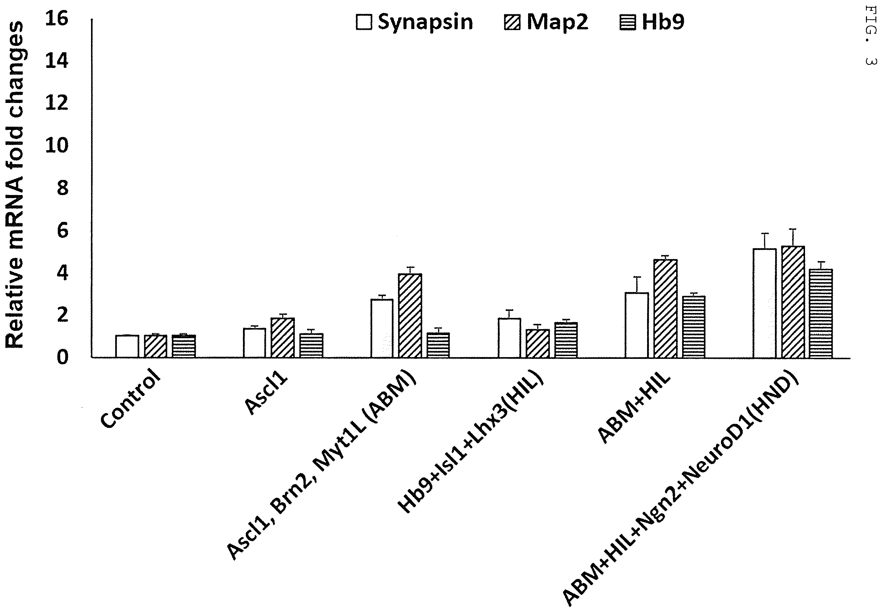

FIG. 3 shows a result of confirming the change in expression of neuronal markers (Synapsin, Map2, and Hb9) to determine the degree of direct cell-conversion into motor neurons when conventional converting factors such as Ascl1, Brn2, Myt1L, Hb9, Isll, Lhx3, Ngn2, and NeuroDl are introduced into human-derived fibroblasts.

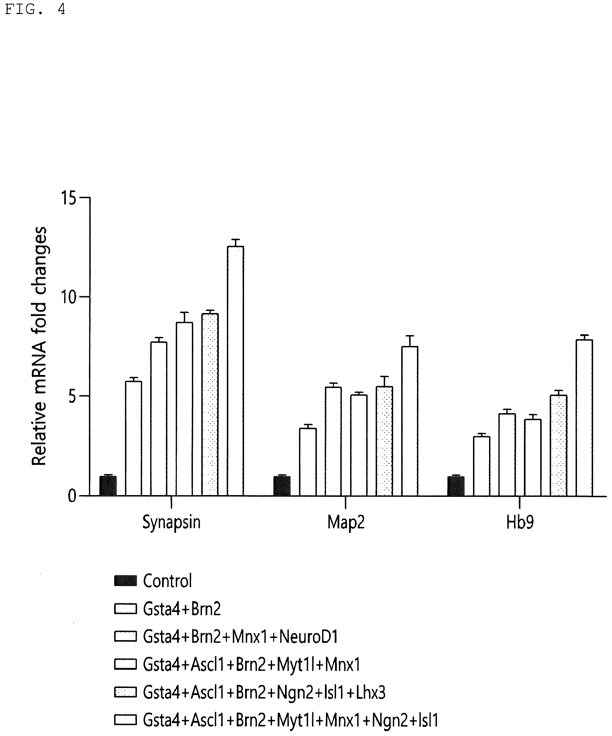

FIG. 4 shows a result of confirming the change in expression of neuronal markers (Synapsin, Map2, and Hb9) to determine the degree of direct cell conversion into motor neurons when the novel converting factor (Gsta4) and conventional converting factors (Ascl1, Brn2, Myt1L, Hb9, Isll, Lhx3, Ngn2, NeuroDl) are introduced into mouse-derived fibroblasts.

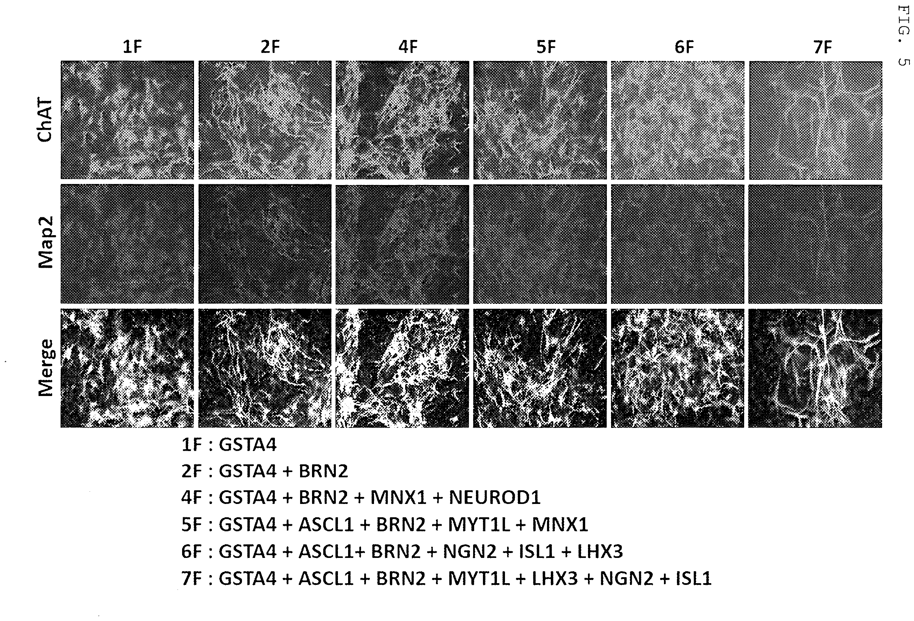

FIG. 5 shows a result of confirming the expression of neuronal markers (ChaT and Map2) by immunofluorescence staining to determine the degree of direct cell-conversion into motor neurons by introducing a converting factor into mouse-derived fibroblasts. The converting factor used in this case is Gsta4 alone or a combination of Gsta4 and a conventional converting factor.

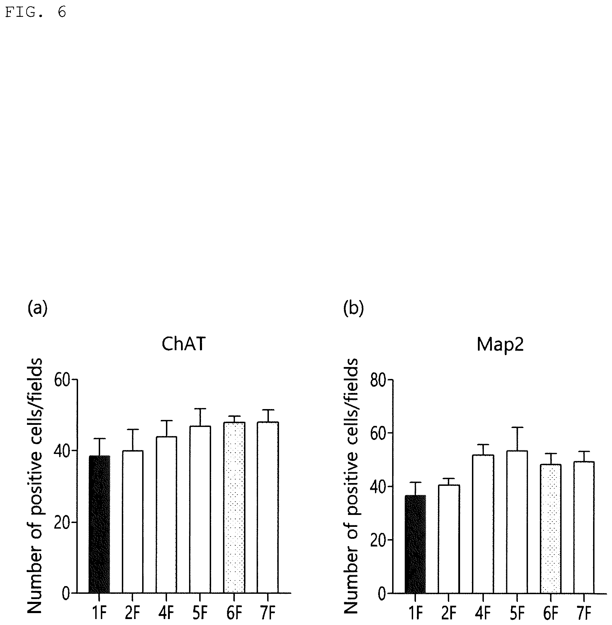

FIG. 6 is a graph showing the number of immunofluorescence-stained cells related to FIG. 5.

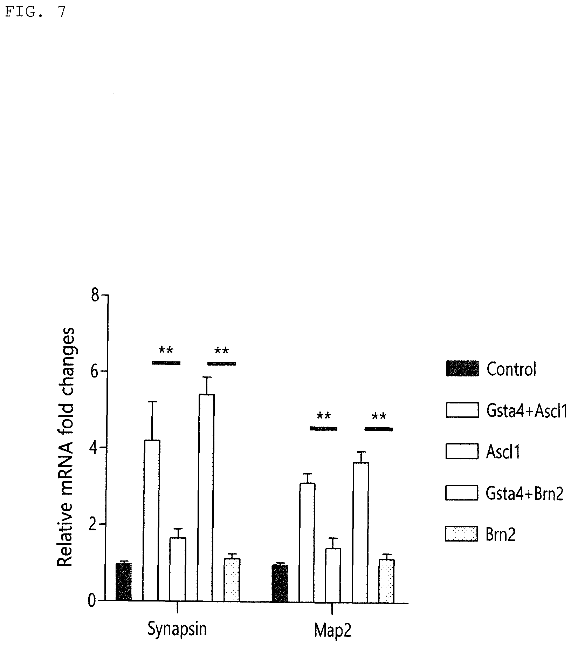

FIG. 7 shows a result of confirming the change in expression of neuronal markers (Synapsin and Map2) to determine the degree of direct cell-conversion into motor neurons when a converting factor is introduced into mouse-derived fibroblasts. The converting factors used in this case are a conventional converting factor alone and a combination of Gsta4 and the conventional converting factor.

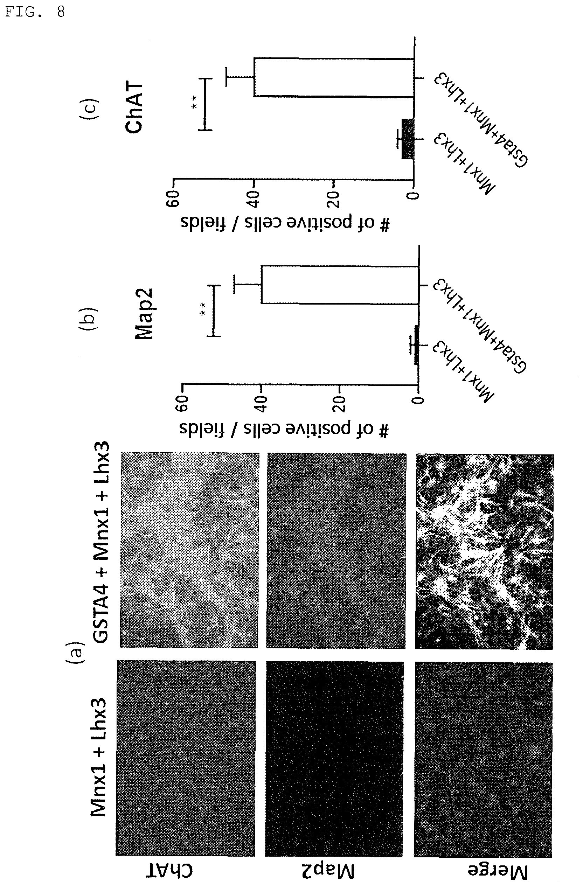

FIG. 8 shows a result of confirming the expression of neuronal markers (ChaT and Map2) by immunofluorescence staining to determine the degree of direct cell-conversion into motor neurons when converting factors are introduced into mouse-derived fibroblasts. The converting factors used in this case are a combination of Mnx1 and Lhx3 and a combination of Gsta4, Mnx1, and Lhx3, in which (a) is the result of immunofluorescence staining, and (b) is the numerical value of the result of (a).

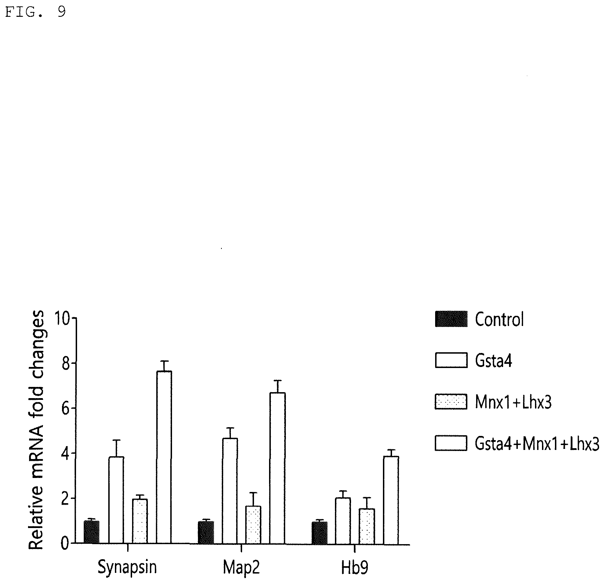

FIG. 9 shows a result of confirming the expression of neuronal markers (Synapsin, Map2, and Hb9) to determine the degree of direct cell-conversion into motor neurons when converting factors are introduced into mouse-derived fibroblasts. The converting factors used in this case are Gsta4 alone, a combination of Mnx1 and Lhx3, and a combination of Gsta4, Mnx1, and Lhx3.

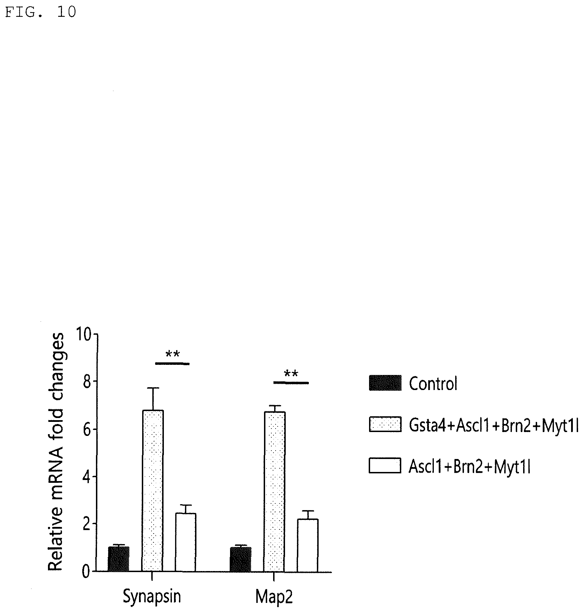

FIG. 10 shows a result of confirming the expression of neuronal markers (Synapsin and Map2) to determine the degree of direct cell-conversion into motor neurons when converting factors are introduced into mouse-derived fibroblasts. The converting factors used in this case are a combination of Ascl1, Brn2 and MytlL and a combination of Gsta4, Ascl1, Brn2, and Myt1L.

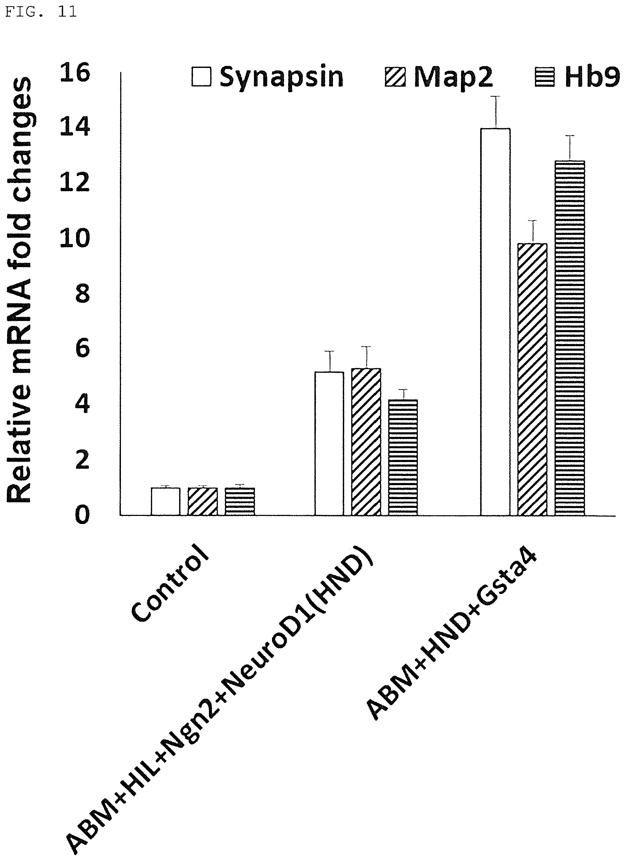

FIG. 11 shows a result of confirming the change in expression of neuronal markers (Synapsin, Map2, and Hb9) to determine the degree of direct cell-conversion into motor neurons when converting factors are introduced into human-derived fibroblasts. The converting factors used in this case are a combination of Ascl1, Brn2, Myt1L, Hb9, Isll, Lhx3, Ngn2 and NeuroDl and a combination of Ascl1, Brn2, Myt1L, Hb9, Isll, Lhx3, Ngn2, NeuroDl, and Gsta4.

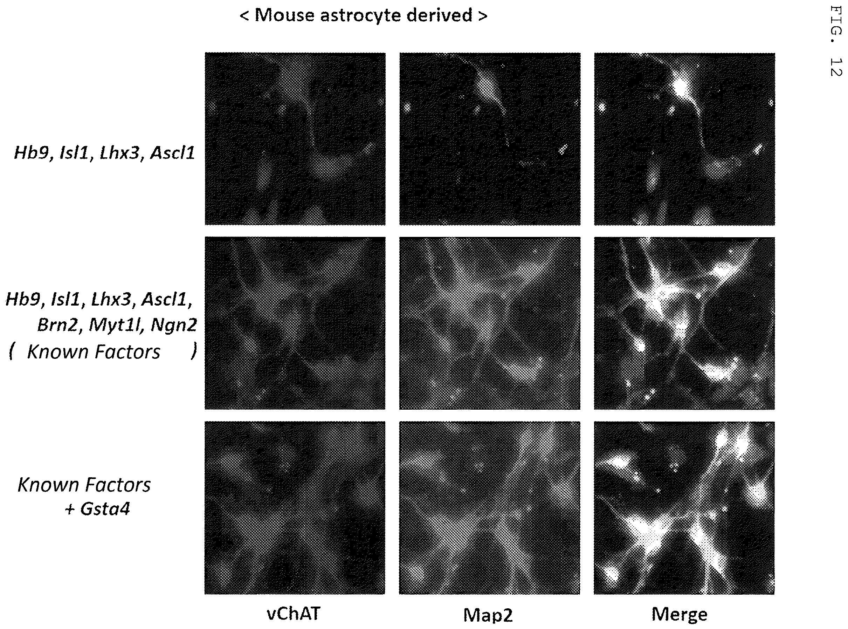

FIG. 12 shows a result of confirming the expression of neuronal markers (vChaT and Map2) by immunofluorescence staining to determine the degree of direct cell-conversion into motor neurons when converting factors are introduced into mouse astrocyte. The converting factors used in this case are a combination of Hb9, Isll, Lhx3 and Ascl1, a combination of Hb9, Isll, Lhx3, Ascl1, Brn2, MytlL and Ngn2 (which will be referred to as conventional converting factors), and a combination of Gsta4 and conventional converting factors.

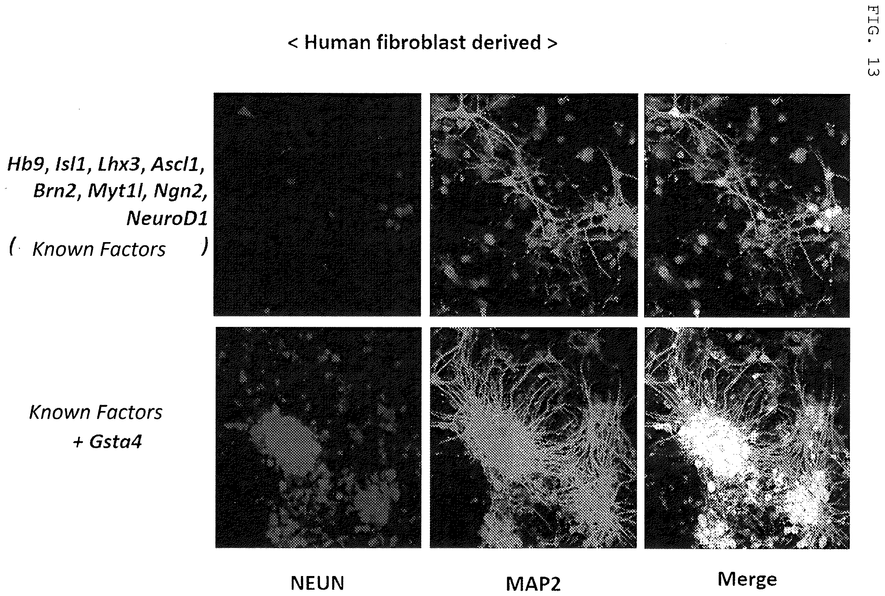

FIG. 13 shows a result of confirming the expression of neuronal markers (NEUN and Map2) by immunofluorescence staining to determine the degree of direct cell-conversion into motor neurons when converting factors are introduced into human-derived fibroblasts.

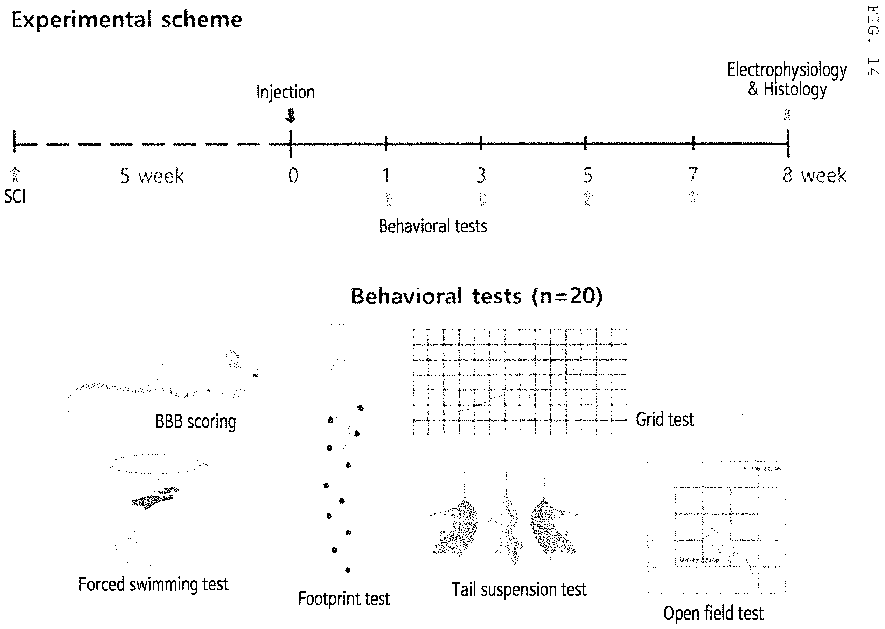

FIG. 14 is a diagram of an overall experimental design showing morphological changes, physiological analysis, and behavioral analysis by injecting converting factors to L5, which is a spinal cord injury site of a spinal cord injury (SCI) mouse model, which is a paraplegic model due to spinal cord injury.

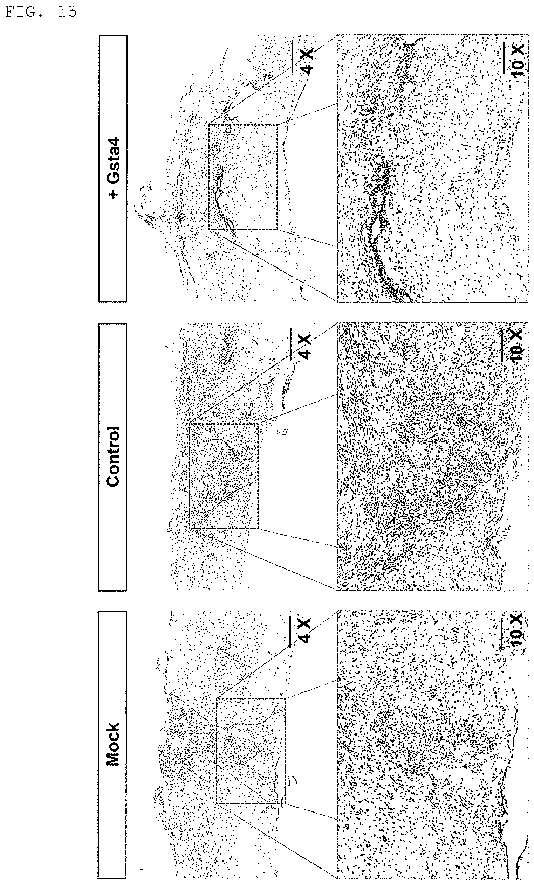

FIG. 15 is a result of crystal violet staining to confirm morphological changes in the spinal cord injury site after injection of converting factors to the SCI mouse model. Here, "mock" refers an AAV injection group in which the subjects were injected with Adeno-associated virus (AAV), "control" refers to an AAV injection group into which conventional converting factors (Ascl1, Brn2, Myt1l, Hb9, Isll, Lhx3, Ngn2, and NeuroDl combination) were introduced, and "+Gsta4" refers to an AAV injection group into which the conventional converting factors and Gsta4 were introduced.

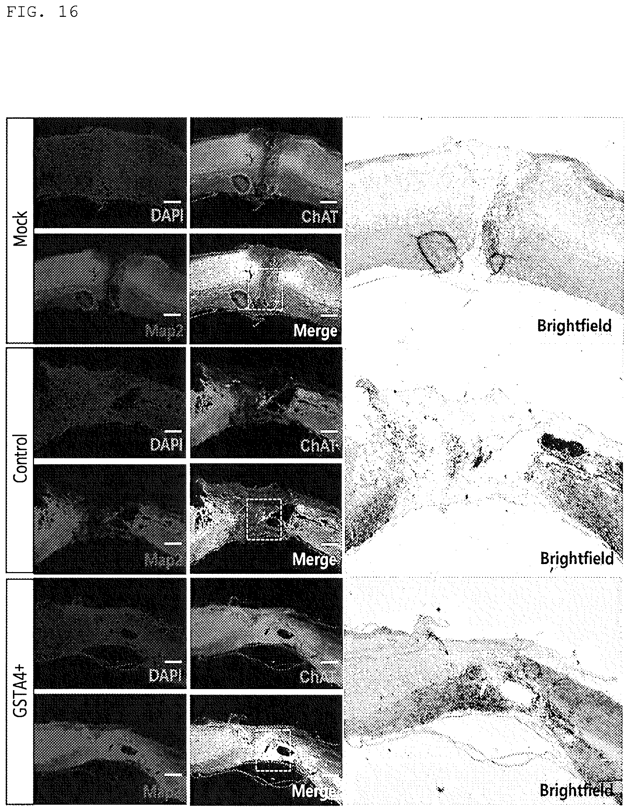

FIG. 16 shows a result of immunofluorescence staining of motor neuron markers (ChaT and Map2) after injection of converting factors into the SCI mouse model. Here, "mock" refers to an AAV injection group in which the subjects were injected with Adeno-associated virus (AAV), "control" refers to an AAV injection group into which conventional converting factors (Ascl1, Brn2, Myt1l, Hb9, Isll, Lhx3, Ngn2, and NeuroDl combination) were introduced, and "Gsta4" refers to an AAV injection group into which the conventional converting factors and Gsta4 were introduced.

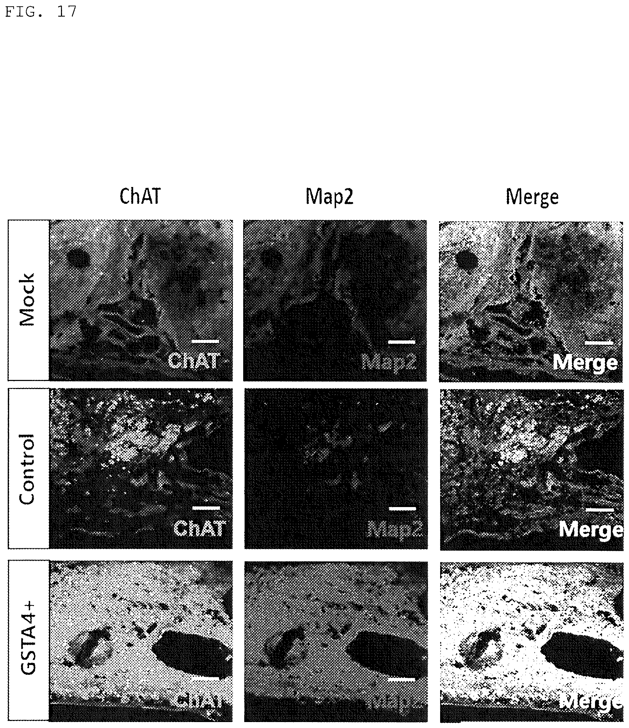

FIG. 17 is a result of checking the square box portion indicated by the white dotted line in FIG. 16.

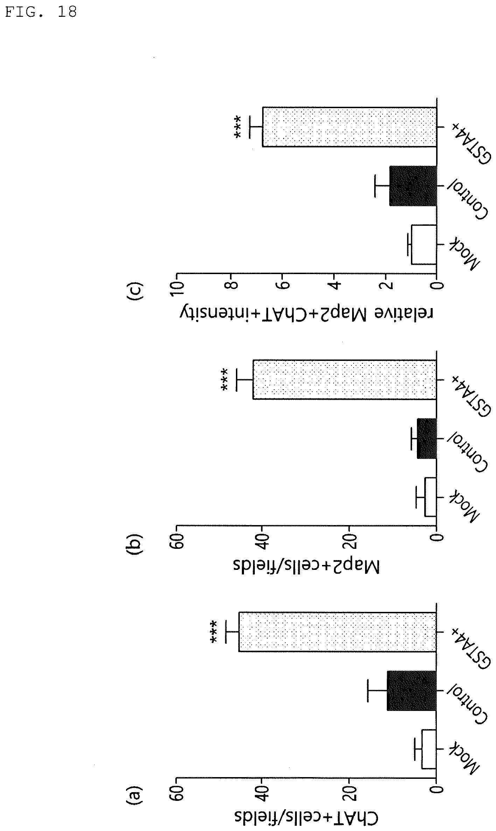

FIG. 18 is a numerical representation of the immunofluorescence staining results of FIG. 16.

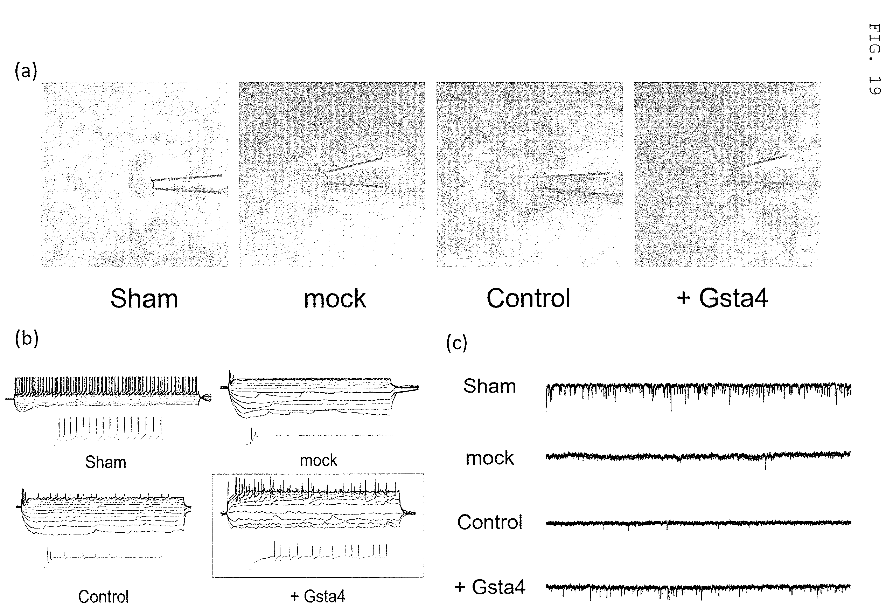

FIG. 19 is a result of analyzing electrophysiological changes of motor neurons after injection of converting factors into an SCI mouse model, in which (a) is a patch-clamped neuron, (b) is a result of measuring an action potential, and (c) is a result of measuring signals coming from pre-synaptic neurons. Here, "Sham" refers to a normal group with no spinal cord damage, "mock" refers an AAV injection group in which the SCI mouse model was injected with AAV, "control" refers to an AAV injection group into which conventional converting factors (Ascl1, Brn2, Myt1l, Hb9, Isll, Lhx3, Ngn2, and NeuroDl combination) were introduced, and "+Gsta4" refers to an AAV injection group into which the conventional converting factors and Gsta4 were introduced.

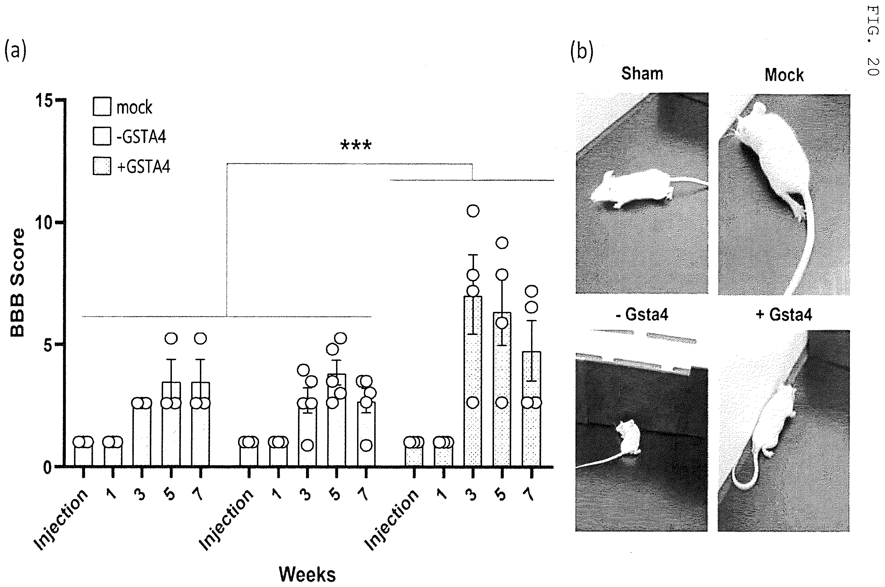

FIG. 20 is a result of analyzing the treatment effect of paraplegia in the spinal cord injury site (L5) by Basso Beattie, and Bresnahan score (BBB score) after injection of converting factors into the SCI mouse model, in which (a) is a graph, which is a numerical representation of the observation results of (b). Here, "Sham" refers to a normal group with no spinal cord damage, "mock" refers an AAV injection group in which the SCI mouse model was injected with AAV, "-Gsta4" refers to an AAV injection group into which conventional converting factors (Ascl1, Brn2, Myt1l, Hb9, Isll, Lhx3, Ngn2, and NeuroDl combination) were introduced, and "+Gsta4" refers to an AAV injection group into which the conventional converting factors and Gsta4 were introduced.

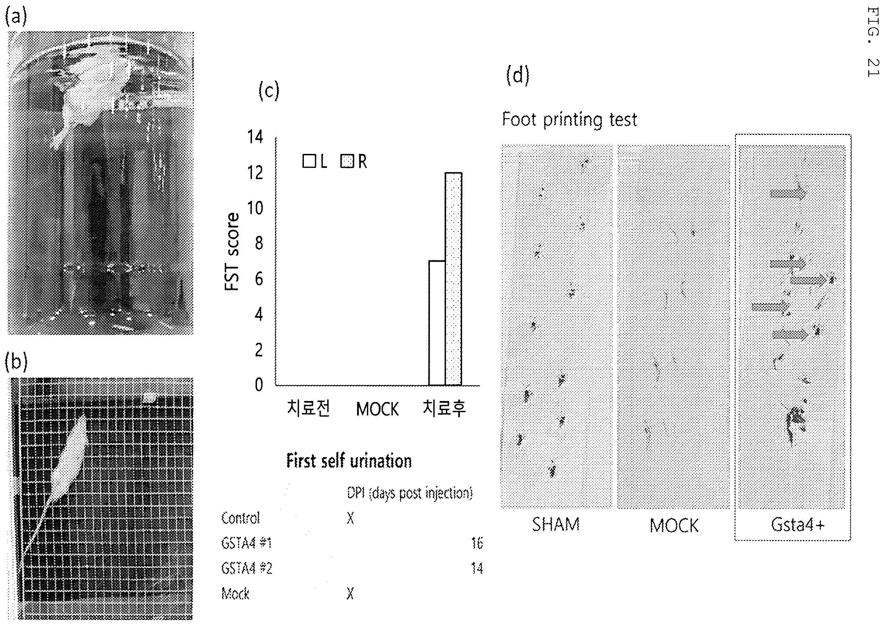

FIG. 21 is a result of confirming the treatment effect of paraplegia by behavioral analysis after injection of a converting factor into the SCI mouse model, in which (a) represents a forced swimming test, (b) represents self-urination, (c) represents a numerical representation of (a) and (b), and (d) represents the result of a foot printing test.

### Best Mode

Unless otherwise defined, all terms including technical and scientific terms used herein have the same meaning as commonly understood by those who are ordinarily skilled in the art to which the present invention pertains. While methods and materials similar or identical to those described herein may be used for execution or testing of the present invention, suitable methods and materials are described below. All publications, patent applications, papers, and other references mentioned herein are incorporated by reference in their entirety. In addition, the materials, methods, and examples are only illustrative and are not intended to be limiting.

### Definition of Terms

### • Direct reprogramming/direct cell-conversion/transdifferentiation

The terms "direct reprogramming, direct conversion, and transdifferentiation" used herein mean that a converting factor is introduced into a differentiated cell to be converted into a desired cell. In particular, direct cell-conversion induces conversion between completely different types of mature cells. In other words, it means conversion from a somatic cell into a desired cell without involving a pluripotent state. The terms "direct reprogramming", "direct cell-conversion", and "transdifferentiation" can be interchangeably used.

### • Direct cell-conversion factor, converting factor

In this application, the term "direct cell-conversion factor" refers to a factor involved in direct cell-conversion. In this case, the converting factor may be a gene, compound, protein, nucleic acid, or the like. The term "direct cell-conversion factor" and the term "converting factor" can be interchangeably used.

### • Induced motor neuron (iMN)

In this application, the term "induced motor neuron" refers to a motor neuron induced by direct cell-conversion. The induced motor neuron (iMN) refers to a motor neuron artificially produced by directly converting a somatic cell, using the novel converting factor of the present application. The term "induced motor neuron", the term "motor neuron", the term "iMN" can be used interchangeably.

### • Treatment

As used herein, the term "treatment" means alleviating or inhibiting the progression of a disease, disorder, symptom, or the like. The treatment refers to all activities in which symptoms are alleviated or beneficially changed by the composition, direct cell-conversion, or the like of the present invention. Substances exhibiting such therapeutic effects are collectively referred to as "treatment agent" or "pharmaceutical composition". In particular, gene therapy refers to a genetic material that transfers various forms of genes into the human body by various methods for the purpose of treating diseases. Alternatively, cell therapy refers to a case in which a therapeutic substance is delivered in the form of cells in the human body for the purpose of treating a disease or the like.

### • About

As used herein, the term "about" refers to a degree close to a certain quantity, and it refers to an amount, level, value, number, frequency, percent, dimension, size, amount, weight, or length that varies to the extent of 30%, 25%, 20%, 15%, 10%, 9%, 8%, 7%, 6%, 5%, 4%, 3%, 2%, or 1% with respect to a reference amount, level, value, number, frequency, percentage, dimension, size, amount, weight, or length.

### 1. Novel direct cell-conversion factor: glutathione S-transferase alpha 4 (Gsta4)

### Characteristics of direct cell-conversion

Direct cell-conversion is to produce a different system cell without involving the reprogramming of a fully differentiated cell into a stem cell. This process has the effect of converting a fully differentiated cell into a specific cell of a different system without via a stem cell stage.

To date, there are two methodologies for direct cell-conversion: somatic cell-specific factor-mediated direct reprogramming (SDR), and pluripotent cell-specific factor-mediated direct reprogramming (PDR). SDR is a method of inducing direct cell-conversion into a target cell by overexpressing a factor that is highly likely to be expressed in the target cell. PDR is a method of dedifferentiating cells into an incomplete intermediate cell type (cells similar to stem cells) using OCT4, SOX2, KLF4, and C-Myc, which are factors used for reprogramming of induced pluripotent stem cells, and then directly converting the intermediate cell type into a target cell type by making a culture medium. In the case of PDR, even when being directly converted into various cell types, four reprogramming factors (OCT4, SOX2, KLF4, and C-Myc) that are initially overexpressed are commonly used.

### Known direct cell-conversion factors

Known converting factors that are being used for direct cell-conversion include Achaete-scute homolog 1 (Ascl1), nuclear receptor subfamily 4 Group A member 2 (Nurr1), LIM homeobox transcription factor 1 alpha (Lmxla), forkhead box protein A2 (Foxa2), POU Class 3 homeobox 2 (Brn2), sex determining region Y-box 2 (Sox2), forkhead box G1 (Foxg1), LIM homeobox 3 (Lhx3), homeobox HB9 (HB9), ISL LIM homeobox 1 (IsL1), neurogenin 2 (Ngn2), neuronal differentiation 1 (NeuroDl), Myelin transcription factor like 1 (Myt1l), neuronal differentiation 2 (NeuroD2), microRNA-9 (miR-9), microRNA-124 (miR-124), Zic family member 1 (Zic1), GATA binding protein 5 (Gata5), hepatocyte nuclear factor 4 alpha (Hnf4α), hepatocyte nuclear factor 1 alpha (HNF1α), forkhead box A1 (Foxa1), forkhead box A3 (Foxa3), T-box transcription factor 5 (Tbx5), myocyte enhancer factor 2C (Mef2c), octamer-binding transcription factor 4 (Oct4), PR/SET domain 16 (PRDM16), myoblast determination protein 1 (MyoD), Kruppel-like factor 4 (Klf4), and cellular Myc (c-Myc) [Mol Cell. 2012 Sep 28;47(6):827-838].

In particular, Ascl1, Brn2, Myt1L, Hb9, Isll, Lhx3, Ngn2, NeuroDl, and the like are known as converting factors used for direct cell-conversion into motor neurons.

### Problems of conventional direct cell-conversion factors

The direct cell-conversion has the advantage of being able to switch between differentiated cells having completely different cell types without via an induced pluripotent stem cell stage. However, the converting factors known to date have had a problem that intercellular conversion efficiency is low or nearly zero when used alone or in combination.

### Overview of novel direct cell-conversion factor Gsta4

The present application discloses a novel direct cell-conversion factor, glutathione S-transferase alpha 4 (Gsta4).

Even with the use of the Gsta4, it is possible to convert somatic cells effectively directly into motor neurons. Furthermore, when the Gsta4 is used in conjunction with a conventional direct cell-conversion factor, the efficiency of cell conversion into motor neurons can be increased compared to the case of using only a conventional converting factor.

Hereinafter, Gsta4, which is a novel converting factor, will be described in more detail.

### Known functions of Gsta4

Gsta4 is an enzyme belonging to glutathione S-transferases (GST). The mammalian cytoplasm is known to produce eight GSTs: alpha, kappa, mu, omega, pi, sigma, theta, and zeta. Among them, alpha is located on chromosome 6 and includes GSTA1, GSTA2, GSTA3, GSTA4, and GSTA5.

Gsta4, also referred to as GTA4, is known to be involved in cell defense mechanisms, toxin degradation, and anticancer effects. In addition, it is known to play an important role in reducing cellular stress by reducing active oxygen. The absence of Gsta4 is known to be negatively related to diseases such as Parkinson's disease and Alzheimer's disease. However, its function as a direct cell-conversion factor has not been reported.

### New function of Gsta4

The present application discloses a novel function of Gsta4, enabling direct cell-conversion. That is, Gsta4 is disclosed as a novel converting factor for direct cell-conversion.

In particular, it has not been previously reported that Gsta4 of the present application has a function of converting somatic cells into motor neurons (direct cell-conversion function), and the present inventors experimentally confirmed and discovered it. The term "Gsta4" described below is interpreted as a member acting as a direct cell-conversion factor, which is a novel function. In addition, herein below, when described as Gsta4 for convenience, it is interpreted as referring to a Gsta4 protein or a nucleic acid encoding the Gsta4 protein, depending on the context.

### Gsta4 type (1) - Gsta4 protein

In one aspect, the present application discloses a Gsta4 protein for direct cell-conversion.

The Gsta4 protein may be wild-type or a variant. The variant may be obtained by deletion, substitution, or insertion of one or more amino acid residues of a wild-type amino acid sequence. The Gsta4 variant may have a sequence with sequence identity to a wild-type amino acid sequence. In this case, the sequence identity may be 50% to 99%. Preferably, the Gsta4 protein may have at least 70%, 75%, 80%, 85%, 90%, 91%, 92%, 93%, 94%, 95%, 96%, 97%, 98%, or 99% sequence identity to the wild-type amino acid sequence.

In any embodiment, the Gsta4 protein for direct conversion of the present application may be the amino acid sequence represented by SEQ ID NO: 1.

SEQ ID NO: 1: maarpklhyp ngrgrmesvr wvlaaagvef deefletkeq lyklqdgnhl lfqqvpmvei dgmklvqtrs ilhyiadkhn lfgknlkert lidmyvegtl dllellimhp flkpddqqke vvnmaqkaii ryfpvfekil rghgqsflvg nqlsladvil lqtilaleek ipnilsafpf lqeytvklsn iptikrflep gskkkpppde iyvrtvynif rp

In another embodiment, the Gsta4 protein may be an amino acid sequence having at least 70%, 75%, 80%, 85%, 90%, 91%, 92%, 93%, 94%, 95%, 96%, 97%, 98%, or 99% sequence identity to the amino acid sequence represented by SEQ ID NO: 1.

### Gsta4 type (2) - Nucleic acid encoding Gsta4 protein

In another example, the present application discloses a nucleic acid encoding a Gsta4 protein for direct cell-conversion. The nucleic acid may be DNA or RNA.

The nucleic acid encoding the Gsta4 protein may be wild-type or a variant. The variant may be obtained by deletion, substitution, or insertion of one or more nucleic acid residues of a wild-type nucleic acid sequence. In addition, the Gsta4 variant may have sequence identity with a nucleic acid sequence encoding a wild-type protein. In this case, the sequence identity may be in a range of from 50% to 99%. Preferably, the nucleic acid encoding the Gsta4 protein may have at least 70%, 75%, 80%, 85%, 90%, 91%, 92%, 93%, 94%, 95%, 96%, 97%, 98%, or 99% sequence identity to the wild-type nucleic acid sequence.

The nucleic acid sequence encoding the Gsta4 protein may be a known sequence.

The nucleic acid sequence may include intron sequences and exon sequences.

Alternatively, the nucleic acid sequence may include only exon sequences.

For example, the nucleic acid sequence encoding the Gsta4 protein may include a coding sequence (CDS) sequence excluding introns.

The nucleic acid sequence may be a DNA sequence encoding the Gsta4 protein and an mRNA sequence (transcription result) corresponding thereto.

The nucleic acid sequence may be an mRNA sequence (transcription result) corresponding to the exon sequence of the Gsta4 gene.

In one embodiment, the nucleic acid encoding the Gsta4 protein may include SEQ ID NO: 2, which is the full DNA sequence of the Gsta4 gene.

SEQ ID NO: 2: gctttgtgcg gctccaggcc tccgagtgga ctccagaaag
cctgaaaagc tatcatggca gcaaggccca agctccacta tcccaacgga agaggccgga
tggagtccgt gagatgggtt ttagctgccg ccggagtcga gtttgatgaa gaatttctgg
aaacaaaaga acagttgtac aagttgcagg atggtaacca cctgctgttc caacaagtgc
ccatggttga aattgacggg atgaagttgg tacagacccg aagcattctc cactacatag
cagacaagca caatctcttt ggcaagaacc tcaaggagag aaccctgatt gacatgtacg
tggaggggac actggatctg ctggaactgc ttatcatgca tcctttctta aaaccagatg

atcagcaaaa ggaagtggtt aacatggccc agaaggctat aattagatac tttcctgtgt
ttgaaaagat tttaaggggt cacggacaaa gctttcttgt tggtaatcag ctgagccttg
cagatgtgat tttactccaa accattttag ctctagaaga gaaaattcct aatatcctgt
ctgcatttcc tttcctccag gaatacacag tgaaactaag taatatccct acaattaaga
gattccttga acctggcagc aagaagaagc ctccccctga tgaaatttat gtgagaaccg
tctacaacat ctttaggcca taaaacaaca catccatgtg tgagtgacag tgtgttccta
gagatggtat tgtctacagt catgtcttaa tggatcccag ctctgtcatg gtgctatcta
tgtattaagt tgggtcctaa gttgggtctt ttgtgtcaac gagatcatct cttctagaaa
tatcaacctt ttttgtccag taaataattg ttaggggatc tttattggaa aacttttttg
gagaggctgg tatttaagtt agatctgatt gggctactca tgtcctgtag ccagttcatc
ctcataataa gaatgggcag gatctcttgt tctctcctga gtgtctttct actctcctga
gcgtctttct gctctcctta tcctgttctc ttatccttat cccctccagt ctctgcctaa
tttttagtgt ttaataacaa ccgaatgtct agtaaatgac tctcctctga gctgtaataa
ataaaatggt agtaatgaat gcaatcagta ttagccaaaa taaagaattt atgagtcatt

In another embodiment, the nucleic acid encoding the Gsta4 protein may have SEQ ID NO: 12, which is a CDS sequence.

SEQ ID NO: 12:
Atggcagcaaggcccaagctccactatcccaacggaagaggccggatggagtccgtgagatgg
gttttagctgccgccggagtcgagtttgatgaagaatttctggaaacaaaagaacagttgtac
aagttgcaggatggtaaccacctgctgttccaacaagtgcccatggttgaaattgacgggatg
aagttggtacagacccgaagcattctccactacatagcagacaagcacaatctctttggcaag
aacctcaaggagagaaccctgattgacatgtacgtggaggggacactggatctgctggaactg
cttatcatgcatcctttcttaaaaccagatgatcagcaaaaggaagtggttaacatggcccag
aaggctataattagatactttcctgtgtttgaaaagattttaaggggtcacggacaaagcttt

cttgttggtaatcagctgagccttgcagatgtgattttactccaaaccattttagctctagaa
gagaaaattcctaatatcctgtctgcatttcctttcctccaggaatacacagtgaaactaagt
aatatccctacaattaagagattccttgaacctggcagcaagaagaagcctccccctgatgaa
atttatgtgagaaccgtctacaacatctttaggccataa

In another example, the nucleic acid encoding the Gsta4 protein may have a sequence having at least 70%, 75%, 80%, 85%, 90%, 91%, 92%, 93%, 94%, 95%, 96%, 97%, 98%, or 99% sequence identity to the DNA sequence represented by SEQ ID NO: 2.

In a further example, the nucleic acid encoding the Gsta4 protein may have a sequence having at least 70%, 75%, 80%, 85%, 90%, 91%, 92%, 93%, 94%, 95%, 96%, 97%, 98%, or 99% sequence identity to the SDS sequence represented by SEQ ID NO: 12.

### Gsta4 characteristic (1)

Gsta4, which is a novel direct cell-conversion of the present application, can directly convert somatic cells into motor neurons. For example, Gsta4 converts fibroblasts into motor neurons. For another example, Gsta4 converts astrocytes into motor neurons.

### Gsta4 characteristic (2)

A problem with conventional converting factors is that the efficiency of direct cell-conversion is low or nearly zero. In contrast, Gsta4, a novel converting factor of the present application, has higher direct cell-conversion efficiency than conventional converting factors. For example, when Gsta4 is used alone, the conversion efficiency may be about 2 to 10 times higher than conventional converting factors.

### Gsta4 characteristic (3)

Gsta4 has a boosting function that increases the conversion efficiency of conventional converting factors having low or nearly zero conversion efficiency. The boosting function refers to a function in which conversion efficiency is significantly increased when a conventional converting factor is used with Gsta4, compared to a case where a conventional converting factor alone is used. For example, when Gsta4 is used with a conventional converting factor, the efficiency of direct cell-conversion may be increased about 2 to 10 times compared to other cases.

### Gsta4 characteristic (4)

Since Gsta4 has the effect of directly deriving motor neurons from somatic cells, it can be used to treat nerve system diseases caused by damage to motor neurons. In addition, the therapeutic effect for nerve system diseases can be improved by using Gsta4 and a conventional direct cell-conversion factor together.

For example, when Gsta4 is injected into a subject for treatment of a nervous system disease, the following advantages can be obtained: reduction in scar caused by inflammatory cells, increase in action potential, and increase in signal transmission between presynaptic neurons.

### 2. Converting factors that can be combined with Gsta4

### Overview of converting factors that can be combined with Gsta4

In one aspect of the present application, a Gsta4 protein, which is the novel direct cell-conversion factor of the present application, or a nucleic acid encoding the Gsta4 protein can be used solely or in combination with a known direct cell-conversion factor (DC factor).

When the Gsta4 protein is used in combination with a conventionally known converting factor, direct cell-conversion efficiency can be improved, and the time required for differentiation during direct cell-conversion can be reduced.

The known converting factors are not limited if they are known to be involved in direct cell-conversion. In addition, variants of conventional converting factors may also be included. The variant may be a wild-type amino acid or nucleic acid sequence whose one or more amino acid or nucleic acid sequences are deleted, substituted, or inserted.

For example, the conventional converting factors may be one or more proteins selected from Ascl1, Brn2, Myt1L, Hb9, Isll, Lhx3, Ngn2, and NeuroDl.

In one embodiment, the present application discloses a combination of Gsta4 and one or more conventional converting factors.

Hereinafter, examples of possible combinations of Gsta4 and conventional converting factors will be described in more detail.

### Combination example (1) of Gsta4 and conventional converting factors

The Gsta4 may be used in combination with one conventional converting factor selected from Ascl1, Brn2, Myt1L, Hb9, Isll, Lhx3, Ngn2 and NeuroDl.

The combination is, for example, selected from among: Gsta4 + Brn2 (referring to a Gsta4 and Brn2 combination); Gsta4 + Ascl1; Gsta4 + Hb9; Gsta4 + Lhx3; Gsta4 + Myt1L; Gsta4 + Isl1; Gsta4 + Ngn2; and Gsta4 + NeuroDl.

### Combination example (2) of Gsta4 and conventional converting factors

The Gsta4 may be used in combination with two conventional converting factors selected from Ascl1, Brn2, Myt1L, Hb9, Isll, Lhx3, Ngn2, and NeuroDl.

The combination is, for example, selected from among: Gsta4+Brn2+Hb9 (referring to a Gsta4, Brn2, HB9 combination); Gsta4+Ascl1+Hb9; Gsta4+Lhx3+Hb9; Gsta4+Ngn2+Hb9; Gsta4+Ngn2+NeuroD1; Gsta4+Lhx3+Ngn2; and Gsta4+Lhx3+NeuroD1.

### Combination example (3) of Gsta4 and conventional converting factor

The Gsta4 may be used in combination with three conventional converting factors selected from Ascl1, Brn2, Myt1L, Hb9, Isll, Lhx3, Ngn2, and NeuroDl.

The combination is, for example, selected from among: Gsta4+Brn2+Hb9+Ascl1 (referring to a Gsta4, Brn2, Hb9, and Ascl1 combination); Gsta4+Brn2+Hb9+NeuroD1; Gsta4+Ascl1+Hb9+NeuroD1; Gsta4+Lhx3+Hb9+NeuroD1; Gsta4+Lhx3+Hb9+Ngn2; Gsta4+Lhx3+Ngn2+NeuroD1; and Gsta4+ Hb9+Ngn2+NeuroD1.

### Combination example (4) of Gsta4 and conventional converting factor

The Gsta4 may be used in combination with four conventional converting factors selected from Ascl1, Brn2, Myt1L, Hb9, Isll, Lhx3, Ngn2, and NeuroDl.

The combination is, for example, selected from among: Gsta4+Brn2+Hb9+Ascl1+Isl1 (referring to a Gsta4, Brn2, Hb9, Ascl1, and Isl1 combination); Gsta4+Ascl1+Brn2+Myt1L+Hb9; Gsta4+Brn2+Hb9+NeuroD1+Isl1; Gsta4+Ascl1+Hb9+NeuroD1+Isl1; Gsta4+Lhx3+Hb9+NeuroD1+Isl1; Gsta4+Lhx3+Hb9+Ngn2+Isl1; Gsta4+Lhx3+Ngn2+NeuroD1+Isl1; and Gsta4+ Hb9+Ngn2+NeuroD1+Isl1.

### Combination example (5) of Gsta4 and conventional converting factor

The Gsta4 may be used in combination with five conventional converting factors selected from Ascl1, Brn2, Myt1L, Hb9, Isll, Lhx3, Ngn2, and NeuroDl.

The combination is, for example, selected from among:
Gsta4+Brn2+Hb9+Ascl1+Isl1+NeuroD1 (referring to a Gsta4, Brn2, Hb9, Ascl1, Isll, and NeuroDl combination);
Gsta4+Ascl1+Brn2+Ngn2+Isl1+Lhx3;
Gsta4+Brn2+Hb9+NeuroD1+Isl1+Lhx3;
Gsta4+Ascl1+Hb9+NeuroD1+Isl1+Lhx3;
Gsta4+Lhx3+Hb9+NeuroD1+Isl1+Ascl1;
Gsta4+Lhx3+Hb9+Ngn2+Isl1+NeuroD1;
Gsta4+Lhx3+Ngn2+NeuroD1+Isl1+Ascl1; and
Gsta4+Hb9+Ngn2+NeuroD1+Isl1+Ascl1.

### Combination example (6) of Gsta4 and conventional converting factors

The Gsta4 may be used in combination with six conventional converting factors selected from Ascl1, Brn2, Myt1L, Hb9, Isll, Lhx3, Ngn2, and NeuroDl.

The combination is, for example, selected from among: Gsta4+Brn2+Hb9+Ascl1+Isl1+NeuroD1+Lhx3 (referring to Gsta4, Brn2, Hb9, Ascl1, Isll, NeuroDl, and Lhx3 combination); Gsta4+Ascl1+Brn2+Ngn2+Isl1+Lhx3+Myt1L; Gsta4+Brn2+Hb9+NeuroD1+Isl1+Lhx3+Ngn2; and Gsta4+Ascl1+Hb9+NeuroD1+Isl1+Lhx3+Ngn2.

### Combination example (7) of Gsta4 and conventional converting factors

The Gsta4 may be used in combination with seven conventional converting factors selected from Ascl1, Brn2, Myt1L, Hb9, Isll, Lhx3, Ngn2, and NeuroDl.

The combination is, for example, selected from among:
Gsta4+Brn2+Myt1L+Hb9+Isl1+Lhx3+Ngn2+NeuroD1 (referring to Gsta4, Brn2, Myt1L, Hb9, Isll, Lhx3, Ngn2, and NeuroDl combination);
Gsta4+Ascl1+Myt1L+Hb9+Isl1+Lhx3+Ngn2+NeuroD1;
Gsta4+Ascl1+Brn2+Hb9+Isl1+Lhx3+Ngn2+NeuroD1;
Gsta4+Ascl1+Brn2+Myt1L+Isl1+Lhx3+Ngn2+NeuroD1;
Gsta4+Ascl1+Brn2+Myt1L+Hb9+Lhx3+Ngn2+NeuroD1;
Gsta4+Ascl1+Brn2+Myt1L+Hb9+Isl1+Ngn2+NeuroD1;
Gsta4+Ascl1+Brn2+Myt1L+Hb9+Isl1+Lhx3+NeuroD1; and
Gsta4+Ascl1+Brn2+Myt1L+Hb9+Isl1+Lhx3+Ngn2.

### Combination example (8) of Gsta4 and conventional converting factors

The Gsta4 may be used in combination with eight conventional converting factors of Ascl1, Brn2, Myt1L, Hb9, Isll, Lhx3, Ngn2, and NeuroDl.

### Combination ratio of Gsta4 and conventional converting factor

As described above, Gsta4 and conventional converting factors can be used in various combinations.

In this case, for direct cell-conversion, Gsta4 and conventional converting factors may be used in the same amounts.

For example, when Gsta4 and one conventional converting factor are used, they can be used at a ratio of 1:1.

As another example, when Gsta4 and two conventional converting factors are used together, they may be used in a ratio of 1:1:1, 2:1:1, 2:1:2, 1:2:1, or the like.

As a further example, when Gsta4 and three conventional converting factors are used together, they may be used in a ratio of 1:1:1:1.

In addition, for direct cell-conversion, Gsta4 and conventional converting factors may be used in different amounts.

For specific example, when Gsta4, Hb9, and Lhx3 are used together, they are used in a ratio of 1:1:1, 2:1:1, 2:1:2, or 1:2:1.

### 3. Gsta4-containing composition

### Overview of Gsta4-containing composition

The present application discloses a composition essentially containing Gsta4, which is a novel converting factor described above.

For example, the composition may contain only Gsta4.

As another example, the composition may include Gsta4 and a conventional converting factor in combination. For another example, the combination examples (1) to (8) described above can be used.

Each converting factor may be an amino acid sequence constituting a protein and may be included in the composition.

Alternatively, each converting factor may be a nucleic acid sequence encoding each protein and may be included in the composition.

As a specific example, the amino acid sequence of the Gsta4 protein may have a sequence represented by SEQ ID NO: 1, and the nucleic acid encoding the Gsta4 protein may have a sequence represented by SEQ ID NO: 2 or SEQ ID NO: 12.

As a specific example, the amino acid sequence of the Ascl1 protein may have a sequence represented by SEQ ID NO: 17, and the nucleic acid encoding the Ascl1 protein may havea sequence represented by SEQ ID NO: 18.

As a specific example, the amino acid sequence of the Brn2 protein may have a sequence represented by SEQ ID NO: 19, and the nucleic acid encoding the Brn2 protein may have a sequence represented by SEQ ID NO: 20.

As a specific example, the amino acid sequence of the MytlL protein may have a sequence represented by SEQ ID NO: 21, and the nucleic acid encoding the MytlL protein may have a sequence represented by SEQ ID NO: 22.

As a specific example, the amino acid sequence of the Hb9 protein may have a sequence represented by SEQ ID NO: 13, and the nucleic acid encoding the Hb9 protein may have a sequence represented by SEQ ID NO: 14.

As a specific example, the amino acid sequence of the Isl1 protein may have a sequence represented by SEQ ID NO: 23, and the nucleic acid encoding the Isl1 protein may have a sequence represented by SEQ ID NO: 24.

As a specific example, the amino acid sequence of the Lhx3 protein may have a sequence represented by SEQ ID NO: 15, and the nucleic acid encoding the Lhx3 protein may have a sequence represented by SEQ ID NO: 16.

As a specific example, the amino acid sequence of the Ngn2 protein may have a sequence represented by SEQ ID NO: 25, and the nucleic acid encoding the Ngn2 protein may have a sequence represented by SEQ ID NO: 26.

As a specific example, the amino acid sequence of the NeuroDl protein may have a sequence represented by SEQ ID NO: 27, and the nucleic acid encoding the NeuroDl protein may have a sequence represented by SEQ ID NO: 28.

Hereinafter, the composition will be described in more detail.

### Configuration example of composition

### Composition (1): containing Gsta4 solely

The composition of the present application may include Gsta4 protein or a nucleic acid encoding the Gsta4 protein.

In addition, the composition of the present application may include a variant of the protein or a variant of the nucleic acid.

### Composition (2): containing Gsta4 + conventional converting factor

The composition of the present application may include:
i) Gsta4 protein or a nucleic acid encoding the Gsta4 protein; and
ii) one protein selected from Ascl1, Brn2, Myt1L, Hb9, Isll, Lhx3, Ngn2, and NeuroDl, or a nucleic acid encoding the selected protein.

For example, the composition may include:
i) Gsta4 protein or a nucleic acid encoding the same; and
ii) Ascl1 protein or a nucleic acid encoding the same.

For example, the composition may include:
i) Gsta4 protein or a nucleic acid encoding the same; and
ii) Brn2 protein or a nucleic acid encoding the same.

For example, the composition may include:
i) Gsta4 protein or a nucleic acid encoding the same; and
ii) MytlL protein or a nucleic acid encoding the same.

For example, the composition may include:
i) Gsta4 protein or a nucleic acid encoding the same; and
ii) Hb9 protein or a nucleic acid encoding the same.

For example, the composition may include:
i) Gsta4 protein or a nucleic acid encoding the same; and
ii) Isl1 protein or a nucleic acid encoding the same. In this case,
for example, the composition may include:
i) Gsta4 protein or a nucleic acid encoding the same; and
ii) Lhx3 protein or a nucleic acid encoding the same.

For example, the composition may include:
i) Gsta4 protein or a nucleic acid encoding the same; and
ii) Ngn2 protein or a nucleic acid encoding the same.

For example, the composition may include:
i) Gsta4 protein or a nucleic acid encoding the same; and
ii) NeuroDl protein or a nucleic acid encoding the same.

### Composition (3): containing Gsta4 + two conventional converting factors

The composition of the present application may include:
i) Gsta4 protein or a nucleic acid encoding the same; and
ii) two proteins selected from Ascl1, Brn2, Myt1L, Hb9, Isll, Lhx3, Ngn2, and NeuroDl, or nucleic acids encoding the respective selected two proteins.

For example, the composition may include:
i) Gsta4 protein or a nucleic acid encoding the same;
ii) Hb9 protein or a nucleic acid encoding the same; and
iii) Lhx3 protein or a nucleic acid encoding the same.

For example, the composition may include:
i) Gsta4 protein or a nucleic acid encoding the same;
ii) Hb9 protein or a nucleic acid encoding the same; and
iii) Ngn2 protein or a nucleic acid encoding the same.

For example, the composition may include:
i) Gsta4 protein or a nucleic acid encoding the same;
ii) Lhx3 protein or a nucleic acid encoding the same; and
iii) Ngn2 protein or a nucleic acid encoding the same.

For example, the composition may include:
i) Gsta4 protein or a nucleic acid encoding the same;
ii) Hb9 protein or a nucleic acid encoding the same; and
iii) Isl1 protein or a nucleic acid encoding the same.

For example, the composition may include:
i) Gsta4 protein or a nucleic acid encoding the same;
ii) Lhx3 protein or a nucleic acid encoding the same; and
iii) Isl1 protein or a nucleic acid encoding the same.

For example, the composition may include:
i) Gsta4 protein or a nucleic acid encoding the same;
ii) Ngn2 protein or a nucleic acid encoding the same; and
iii) Isl1 protein or a nucleic acid encoding the same.

For example, the composition may include:
i) Gsta4 protein or a nucleic acid encoding the same;
ii) Hb9 protein or a nucleic acid encoding the same; and
iii) Ascl1 protein or a nucleic acid encoding the same.

For example, the composition may include:
i) Gsta4 protein or a nucleic acid encoding the same;
ii) Lhx3 protein or a nucleic acid encoding the same; and
iii) Ascl1 protein or a nucleic acid encoding the same.

For example, the composition may include:
i) Gsta4 protein or a nucleic acid encoding the same;
ii) Ngn2 protein or a nucleic acid encoding the same; and
iii) Ascl1 protein or a nucleic acid encoding the same.

### Composition (4): containing Gsta4 + three conventional converting factors

The composition of the present application may include:
i) Gsta4 protein or a nucleic acid encoding the same; and
ii) three proteins selected from Ascl1, Brn2, Myt1L, Hb9, Isll, Lhx3, Ngn2, and NeuroDl, or nucleic acid encoding the respective selected three proteins.

For example, the composition may include:
i) Gsta4 protein or a nucleic acid encoding the same;
ii) Brn2 protein or a nucleic acid encoding the same;
iii) Hb9 protein or a nucleic acid encoding the same; and
iv) NeuroDl protein or a nucleic acid encoding the same.

For example, the composition may include:
i) Gsta4 protein or a nucleic acid encoding the same;
ii) Hb9 protein or a nucleic acid encoding the same;
iii) Lhx3 protein or a nucleic acid encoding the same; and
iv) Ngn2 protein or a nucleic acid encoding the same.

For example, the composition may include:
i) Gsta4 protein or a nucleic acid encoding the same;
ii) Hb9 protein or a nucleic acid encoding the same;
iii) Lhx3 protein or a nucleic acid encoding the same; and
iv) Isl1 protein or a nucleic acid encoding the same.

For example, the composition may include:
i) Gsta4 protein or a nucleic acid encoding the same;
ii) Hb9 protein or a nucleic acid encoding the same;
iii) Lhx3 protein or a nucleic acid encoding the same; and
iv) Ascl1 protein or a nucleic acid encoding the same.

For example, the composition may include:
i) Gsta4 protein or a nucleic acid encoding the same;
ii) Hb9 protein or a nucleic acid encoding the same;
iii) Lhx3 protein or a nucleic acid encoding the same; and
iv) Brn2 protein or a nucleic acid encoding the same.

For example, the composition may include:
i) Gsta4 protein or a nucleic acid encoding the same;
ii) Hb9 protein or a nucleic acid encoding the same;
iii) Lhx3 protein or a nucleic acid encoding the same; and
iv) Myt1L protein or a nucleic acid encoding the same.

For example, the composition may include:
i) Gsta4 protein or a nucleic acid encoding the same;
ii) Hb9 protein or a nucleic acid encoding the same;
iii) Lhx3 protein or a nucleic acid encoding the same; and
iv) NeuroDl protein or a nucleic acid encoding the same.

For example, the composition may include:
i) Gsta4 protein or a nucleic acid encoding the same;
ii) Hb9 protein or a nucleic acid encoding the same;
iii) Ngn2 protein or a nucleic acid encoding the same; and
iv) Isl1 protein or a nucleic acid encoding the same.

For example, the composition may include:
i) Gsta4 protein or a nucleic acid encoding the same;
ii) Hb9 protein or a nucleic acid encoding the same;
iii) Ngn2 protein or a nucleic acid encoding the same; and
iv) Ascl1 protein or a nucleic acid encoding the same.

For example, the composition may include:
i) Gsta4 protein or a nucleic acid encoding the same;
ii) Lhx3 protein or a nucleic acid encoding the same;
iii) Ngn2 protein or a nucleic acid encoding the same; and
iv) Isl1 protein or a nucleic acid encoding the same.

For example, the composition may include:
i) Gsta4 protein or a nucleic acid encoding the same;
ii) Lhx3 protein or a nucleic acid encoding the same;
iii) Ngn2 protein or a nucleic acid encoding the same; and
iv) Ascl1 protein or a nucleic acid encoding the same.

### Composition (5): containing Gsta4 + four conventional converting factors

The composition of the present application may include:
i) Gsta4 protein or a nucleic acid encoding the Gsta4 protein; and
ii) four proteins selected from Ascl1, Brn2, Myt1L, Hb9, Isll, Lhx3, Ngn2, and NeuroDl, or nucleic acids encoding the respective selected four proteins.

For example, the composition may include:
i) Gsta4 protein or a nucleic acid encoding the same;
ii) Ascl1 protein or a nucleic acid encoding the same;
iii) Brn2 protein or a nucleic acid encoding the same;
iv) MytlL protein or a nucleic acid encoding the same; and
v) Hb9 protein or a nucleic acid encoding the same.

For example, the composition may include:
i) Gsta4 protein or a nucleic acid encoding the same;
ii) Hb9 protein or a nucleic acid encoding the same;
iii) Lhx3 protein or a nucleic acid encoding the same;
iv) Ngn2 protein or a nucleic acid encoding the same; and
v) Isl1 protein or a nucleic acid encoding the same.

For example, the composition may include:
i) Gsta4 protein or a nucleic acid encoding the same;
ii) Hb9 protein or a nucleic acid encoding the same;
iii) Lhx3 protein or a nucleic acid encoding the same;
iv) Ngn2 protein or a nucleic acid encoding the same; and
v) Ascl1 protein or a nucleic acid encoding the same.

For example, the composition may include:
i) Gsta4 protein or a nucleic acid encoding the same;
ii) Hb9 protein or a nucleic acid encoding the same;
iii) Lhx3 protein or a nucleic acid encoding the same;
iv) Ngn2 protein or a nucleic acid encoding the same; and
v) Brn2 protein or a nucleic acid encoding the same.

For example, the composition may include:
i) Gsta4 protein or a nucleic acid encoding the same;
ii) Hb9 protein or a nucleic acid encoding the same;
iii) Lhx3 protein or a nucleic acid encoding the same;
iv) Ngn2 protein or a nucleic acid encoding the same; and
v) MytlL protein or a nucleic acid encoding the same.

For example, the composition may include:
i) Gsta4 protein or a nucleic acid encoding the same;
ii) Hb9 protein or a nucleic acid encoding the same;
iii) Lhx3 protein or a nucleic acid encoding the same;
iv) Ngn2 protein or a nucleic acid encoding the same; and
v) NeuroDl protein or a nucleic acid encoding the same.

For example, the composition may include:
i) Gsta4 protein or a nucleic acid encoding the same;
ii) Lhx3 protein or a nucleic acid encoding the same;
iii) Ngn2 protein or a nucleic acid encoding the same;
iv) Isl1 protein or a nucleic acid encoding the same; and
v) Ascl1 protein or a nucleic acid encoding the same.

For example, the composition may include:
i) Gsta4 protein or a nucleic acid encoding the same;
ii) Lhx3 protein or a nucleic acid encoding the same;
iii) Ngn2 protein or a nucleic acid encoding the same;
iv) Ascl1 protein or a nucleic acid encoding the same; and
v) Brn2 protein or a nucleic acid encoding the same.

For example, the composition may include:
i) Gsta4 protein or a nucleic acid encoding the same;
ii) Hb9 protein or a nucleic acid encoding the same;
iii) Lhx3 protein or a nucleic acid encoding the same;
iv) Isl1 protein or a nucleic acid encoding the same; and
v) Ascl1 protein or a nucleic acid encoding the same.

For example, the composition may include:
i) Gsta4 protein or a nucleic acid encoding the same;
ii) Hb9 protein or a nucleic acid encoding the same;
iii) Lhx3 protein or a nucleic acid encoding the same;
iv) Ascl1 protein or a nucleic acid encoding the same; and
v) Brn2 protein or a nucleic acid encoding the same.

### Composition (6): containing Gsta4 + five conventional converting factors

The composition of the present application may include:
i) Gsta4 protein or a nucleic acid encoding the same; and
ii) five proteins selected from Ascl1, Brn2, Myt1L, Hb9, Isll, Lhx3, Ngn2, and NeuroDl, or nucleic acids encoding the respective selected five proteins.

For example, the composition may include:
i) Gsta4 protein or a nucleic acid encoding the same;
ii) Ascl1 protein or a nucleic acid encoding the same;
iii) Brn2 protein or a nucleic acid encoding the same;
iv) Ngn2 protein or a nucleic acid encoding the same;
v) Isl1 protein or a nucleic acid encoding the same; and
vi) Lhx3 protein or a nucleic acid encoding the same.

For example, the composition may include:
i) Gsta4 protein or a nucleic acid encoding the same;
ii) Hb9 protein or a nucleic acid encoding the same;
iii) Lhx3 protein or a nucleic acid encoding the same;
iv) Ngn2 protein or a nucleic acid encoding the same;
v) Isl1 protein or a nucleic acid encoding the same; and
vi) Ascl1 protein or a nucleic acid encoding the same.

For example, the composition may include:
i) Gsta4 protein or a nucleic acid encoding the same;
ii) Hb9 protein or a nucleic acid encoding the same;
iii) Lhx3 protein or a nucleic acid encoding the same;
iv) Ngn2 protein or a nucleic acid encoding the same;
v) Isl1 protein or a nucleic acid encoding the same; and
vi) Brn2 protein or a nucleic acid encoding the same.

For example, the composition may include:
i) Gsta4 protein or a nucleic acid encoding the same;
ii) Hb9 protein or a nucleic acid encoding the same;
iii) Lhx3 protein or a nucleic acid encoding the same;
iv) Ngn2 protein or a nucleic acid encoding the same;
v) Isl1 protein or a nucleic acid encoding the same; and
vi) MytlL protein or a nucleic acid encoding the same.

For example, the composition may include:
i) Gsta4 protein or a nucleic acid encoding the same;
ii) Hb9 protein or a nucleic acid encoding the same;
iii) Lhx3 protein or a nucleic acid encoding the same;
iv) Ngn2 protein or a nucleic acid encoding the same;
v) Isl1 protein or a nucleic acid encoding the same; and
vi) NeuroDl protein or a nucleic acid encoding the same.

For example, the composition may include:
i) Gsta4 protein or a nucleic acid encoding the same;
ii) Hb9 protein or a nucleic acid encoding the same;
iii) Lhx3 protein or a nucleic acid encoding the same;
iv) Ngn2 protein or a nucleic acid encoding the same;
v) Ascl1 protein or a nucleic acid encoding the same; and
vi) Brn2 protein or a nucleic acid encoding the same.

For example, the composition may include:
i) Gsta4 protein or a nucleic acid encoding the same;
ii) Hb9 protein or a nucleic acid encoding the same;
iii) Lhx3 protein or a nucleic acid encoding the same;
iv) Ngn2 protein or a nucleic acid encoding the same;
v) Ascl1 protein or a nucleic acid encoding the same; and
vi) MytlL protein or a nucleic acid encoding the same.

For example, the composition may include:
i) Gsta4 protein or a nucleic acid encoding the same;
ii) Hb9 protein or a nucleic acid encoding the same;
iii) Lhx3 protein or a nucleic acid encoding the same;
iv) Ngn2 protein or a nucleic acid encoding the same;
v) Ascl1 protein or a nucleic acid encoding the same; and
vi) NeuroDl protein or a nucleic acid encoding the same.

For example, the composition may include:
i) Gsta4 protein or a nucleic acid encoding the same;
ii) Hb9 protein or a nucleic acid encoding the same;
iii) Lhx3 protein or a nucleic acid encoding the same;
iv) Isl1 protein or a nucleic acid encoding the same;
v) Ascl1 protein or a nucleic acid encoding the same; and
vi) Brn2 protein or a nucleic acid encoding the same.

For example, the composition may include:
i) Gsta4 protein or a nucleic acid encoding the same;
ii) Hb9 protein or a nucleic acid encoding the same;
iii) Lhx3 protein or a nucleic acid encoding the same;
iv) Isl1 protein or a nucleic acid encoding the same;
v) Ascl1 protein or a nucleic acid encoding the same; and
vi) MytlL protein or a nucleic acid encoding the same.

For example, the composition may include:
i) Gsta4 protein or a nucleic acid encoding the same;
ii) Hb9 protein or a nucleic acid encoding the same;
iii) Lhx3 protein or a nucleic acid encoding the same;
iv) Isl1 protein or a nucleic acid encoding the same;
v) Ascl1 protein or a nucleic acid encoding the same; and
vi) NeuroDl protein or a nucleic acid encoding the same.

For example, the composition may include:
i) Gsta4 protein or a nucleic acid encoding the same;
ii) Hb9 protein or a nucleic acid encoding the same;
iii) Ngn2 protein or a nucleic acid encoding the same;
iv) Isl1 protein or a nucleic acid encoding the same;
v) Ascl1 protein or a nucleic acid encoding the same; and
vi) Brn2 protein or a nucleic acid encoding the same.

For example, the composition may include:
i) Gsta4 protein or a nucleic acid encoding the same;
ii) Hb9 protein or a nucleic acid encoding the same;
iii) Ngn2 protein or a nucleic acid encoding the same;
iv) Isl1 protein or a nucleic acid encoding the same;
v) Ascl1 protein or a nucleic acid encoding the same; and
vi) MytlL protein or a nucleic acid encoding the same.

For example, the composition may include:
i) Gsta4 protein or a nucleic acid encoding the same;
ii) Hb9 protein or a nucleic acid encoding the same;
iii) Ngn2 protein or a nucleic acid encoding the same;
iv) Isl1 protein or a nucleic acid encoding the same;
v) Ascl1 protein or a nucleic acid encoding the same; and
vi) NeuroDl protein or a nucleic acid encoding the same.

For example, the composition may include:
i) Gsta4 protein or a nucleic acid encoding the same;
ii) Lhx3 protein or a nucleic acid encoding the same;
iii) Ngn2 protein or a nucleic acid encoding the same;
iv) Isl1 protein or a nucleic acid encoding the same;
v) Ascl1 protein or a nucleic acid encoding the same; and
vi) Brn2 protein or a nucleic acid encoding the same.

For example, the composition may include:
i) Gsta4 protein or a nucleic acid encoding the same;
ii) Lhx3 protein or a nucleic acid encoding the same;
iii) Ngn2 protein or a nucleic acid encoding the same;
iv) Isl1 protein or a nucleic acid encoding the same;
v) Ascl1 protein or a nucleic acid encoding the same; and
vi) MytlL protein or a nucleic acid encoding the same.

For example, the composition may include:
i) Gsta4 protein or a nucleic acid encoding the same;
ii) Lhx3 protein or a nucleic acid encoding the same;
iii) Ngn2 protein or a nucleic acid encoding the same;
iv) Isl1 protein or a nucleic acid encoding the same;
v) Ascl1 protein or a nucleic acid encoding the same; and
vi) NeuroDl protein or a nucleic acid encoding the same.

### Composition (7): containing Gsta4 + six conventional converting factors

The composition of the present application may include:
i) Gsta4 protein or a nucleic acid encoding the same; and
ii) six proteins selected from Ascl1, Brn2, Myt1L, Hb9, Isl1, Lhx3, Ngn2, and NeuroD1, or nucleic acids encoding the respective selected six proteins.

For example, the composition may include:
i) Gsta4 protein or a nucleic acid encoding the same;
ii) Ascl1 protein or a nucleic acid encoding the same;
iii) Brn2 protein or a nucleic acid encoding the same;
iv) Ngn2 protein or a nucleic acid encoding the same;
v) Isl1 protein or a nucleic acid encoding the same;
vi) Lhx3 protein or a nucleic acid encoding the same; and
vii) Myt1L protein or a nucleic acid encoding the same.

For example, the composition may include:
i) Gsta4 protein or a nucleic acid encoding the same;
ii) Hb9 protein or a nucleic acid encoding the same;
iii) Lhx3 protein or a nucleic acid encoding the same;
iv) Ngn2 protein or a nucleic acid encoding the same;
v) Isl1 protein or a nucleic acid encoding the same;
vi) Ascl1 protein or a nucleic acid encoding the same; and
vii) Brn2 protein or a nucleic acid encoding the same.

For example, the composition may include:
i) Gsta4 protein or a nucleic acid encoding the same;
ii) Hb9 protein or a nucleic acid encoding the same;
iii) Lhx3 protein or a nucleic acid encoding the same;
iv) Ngn2 protein or a nucleic acid encoding the same;
v) Isl1 protein or a nucleic acid encoding the same;
vi) Ascl1 protein or a nucleic acid encoding the same; and
vii) MytlL protein or a nucleic acid encoding the same.

For example, the composition may include:
i) Gsta4 protein or a nucleic acid encoding the same;
ii) Hb9 protein or a nucleic acid encoding the same;
iii) Lhx3 protein or a nucleic acid encoding the same;
iv) Ngn2 protein or a nucleic acid encoding the same;
v) Isl1 protein or a nucleic acid encoding the same;
vi) Ascl1 protein or a nucleic acid encoding the same; and
vii) NeuroD1 protein or a nucleic acid encoding the same.

For example, the composition may include:
i) Gsta4 protein or a nucleic acid encoding the same;
ii) Hb9 protein or a nucleic acid encoding the same;
iii) Lhx3 protein or a nucleic acid encoding the same;
iv) Ngn2 protein or a nucleic acid encoding the same;
v) Ascl1 protein or a nucleic acid encoding the same;
vi) Brn2 protein or a nucleic acid encoding the same; and
vii) MytlL protein or a nucleic acid encoding the same.

For example, the composition may include:
i) Gsta4 protein or a nucleic acid encoding the same;
ii) Hb9 protein or a nucleic acid encoding the same;
iii) Lhx3 protein or a nucleic acid encoding the same;
iv) Ngn2 protein or a nucleic acid encoding the same;
v) Ascl1 protein or a nucleic acid encoding the same;
vi) Brn2 protein or a nucleic acid encoding the same; and
vii) NeuroD1 protein or a nucleic acid encoding the same.

For example, the composition may include:
i) Gsta4 protein or a nucleic acid encoding the same;
ii) Hb9 protein or a nucleic acid encoding the same;
iii) Lhx3 protein or a nucleic acid encoding the same;
iv) Ngn2 protein or a nucleic acid encoding the same;
v) Isl1 protein or a nucleic acid encoding the same;
vi) Brn2 protein or a nucleic acid encoding the same; and
vii) NeuroD1 protein or a nucleic acid encoding the same.

For example, the composition may include:
i) Gsta4 protein or a nucleic acid encoding the same;
ii) Hb9 protein or a nucleic acid encoding the same;
iii) Lhx3 protein or a nucleic acid encoding the same;
iv) Isl1 protein or a nucleic acid encoding the same;
v) Ascl1 protein or a nucleic acid encoding the same;
vi) Brn2 protein or a nucleic acid encoding the same; and
vii) MytlL protein or a nucleic acid encoding the same.

For example, the composition may include:
i) Gsta4 protein or a nucleic acid encoding the same;
ii) Hb9 protein or a nucleic acid encoding the same;
iii) Lhx3 protein or a nucleic acid encoding the same;
iv) Isl1 protein or a nucleic acid encoding the same;
v) Ascl1 protein or a nucleic acid encoding the same;
vi) Brn2 protein or a nucleic acid encoding the same; and
vii) NeuroD1 protein or a nucleic acid encoding the same.

For example, the composition may include:
i) Gsta4 protein or a nucleic acid encoding the same;
ii) Hb9 protein or a nucleic acid encoding the same;
iii) Ngn2 protein or a nucleic acid encoding the same;
iv) Isl1 protein or a nucleic acid encoding the same;
v) Ascl1 protein or a nucleic acid encoding the same;
vi) Brn2 protein or a nucleic acid encoding the same; and
vii) Myt1L protein or a nucleic acid encoding the same.

For example, the composition may include:
i) Gsta4 protein or a nucleic acid encoding the same;
ii) Hb9 protein or a nucleic acid encoding the same;
iii) Ngn2 protein or a nucleic acid encoding the same;
iv) Isl1 protein or a nucleic acid encoding the same;
v) Ascl1 protein or a nucleic acid encoding the same;
vi) Brn2 protein or a nucleic acid encoding the same; and
vii) NeuroD1 protein or a nucleic acid encoding the same.

For example, the composition may include:
i) Gsta4 protein or a nucleic acid encoding the same;
ii) Lhx3 protein or a nucleic acid encoding the same;
iii) Ngn2 protein or a nucleic acid encoding the same;
iv) Isl1 protein or a nucleic acid encoding the same;
v) Ascl1 protein or a nucleic acid encoding the same;
vi) Brn2 protein or a nucleic acid encoding the same; and
vii) MytlL protein or a nucleic acid encoding the same.

For example, the composition may include:
i) Gsta4 protein or a nucleic acid encoding the same;
ii) Lhx3 protein or a nucleic acid encoding the same;
iii) Ngn2 protein or a nucleic acid encoding the same;
iv) Isl1 protein or a nucleic acid encoding the same;
v) Ascl1 protein or a nucleic acid encoding the same;
vi) Brn2 protein or a nucleic acid encoding the same; and
vii) NeuroD1 protein or a nucleic acid encoding the same.

### Composition (8): containing Gsta4 + seven conventional converting factors

The composition of the present application may include:
i) Gsta4 protein or a nucleic acid encoding the same; and
ii) seven proteins selected from Ascl1, Brn2, Myt1L, Hb9, Isl1, Lhx3, Ngn2, and NeuroD1, or nucleic acids encoding the respective selected seven proteins.

For example, the composition may include:
i) Gsta4 protein or a nucleic acid encoding the same;
ii) Brn2 protein or a nucleic acid encoding the same;
iii) MytlL protein or a nucleic acid encoding the same;
iv) Hb9 protein or a nucleic acid encoding the same;
v) Isl1 protein or a nucleic acid encoding the same;
vi) Lhx3 protein or a nucleic acid encoding the same;
vii) Ngn2 protein or a nucleic acid encoding the Ngn2 protein; and
viii) Ascl1 protein or a nucleic acid encoding the same.

For example, the composition may include:
i) Gsta4 protein or a nucleic acid encoding the same;
ii) Hb9 protein or a nucleic acid encoding the same;
iii) Lhx3 protein or a nucleic acid encoding the same;
iv) Ngn2 protein or a nucleic acid encoding the same;
v) Isl1 protein or a nucleic acid encoding the same;
vi) Ascl1 protein or a nucleic acid encoding the same;
vii) Brn2 protein or a nucleic acid encoding the Ngn2 protein; and
viii) MytlL protein or a nucleic acid encoding the same.

For example, the composition may include:
i) Gsta4 protein or a nucleic acid encoding the same;
ii) Hb9 protein or a nucleic acid encoding the same;
iii) Lhx3 protein or a nucleic acid encoding the same;
iv) Ngn2 protein or a nucleic acid encoding the same;
v) Isl1 protein or a nucleic acid encoding the same;
vi) Ascl1 protein or a nucleic acid encoding the same;
vii) Brn2 protein or a nucleic acid encoding the Ngn2 protein; and
viii) NeuroD1 protein or a nucleic acid encoding the same.

For example, the composition may include:
i) Gsta4 protein or a nucleic acid encoding the same;
ii) Hb9 protein or a nucleic acid encoding the same;
iii) Lhx3 protein or a nucleic acid encoding the same;
iv) Ngn2 protein or a nucleic acid encoding the same;
v) Ascl1 protein or a nucleic acid encoding the same;
vi) Brn2 protein or a nucleic acid encoding the same;
vii) MytlL protein or a nucleic acid encoding the Ngn2 protein; and
viii) NeuroD1 protein or a nucleic acid encoding the same.

For example, the composition may include:
i) Gsta4 protein or a nucleic acid encoding the same;
ii) Hb9 protein or a nucleic acid encoding the same;
iii) Lhx3 protein or a nucleic acid encoding the same;
iv) Ngn2 protein or a nucleic acid encoding the same;
v) Isl1 protein or a nucleic acid encoding the same;
vi) Brn2 protein or a nucleic acid encoding the same;
vii) MytlL protein or a nucleic acid encoding the Ngn2 protein; and
viii) NeuroD1 protein or a nucleic acid encoding the same.

For example, the composition may include:
i) Gsta4 protein or a nucleic acid encoding the same;
ii) Hb9 protein or a nucleic acid encoding the same;
iii) Lhx3 protein or a nucleic acid encoding the same;
iv) Ngn2 protein or a nucleic acid encoding the same;
v) Isl1 protein or a nucleic acid encoding the same;
vi) Ascl1 protein or a nucleic acid encoding the same;
vii) MytlL protein or a nucleic acid encoding the Ngn2 protein; and
viii) NeuroD1 protein or a nucleic acid encoding the same.

For example, the composition may include:
i) Gsta4 protein or a nucleic acid encoding the same;
ii) Hb9 protein or a nucleic acid encoding the same;
iii) Lhx3 protein or a nucleic acid encoding the same;
iv) Ngn2 protein or a nucleic acid encoding the same;
v) Isl1 protein or a nucleic acid encoding the same;
vi) Ascl1 protein or a nucleic acid encoding the same;
vii) Brn2 protein or a nucleic acid encoding the Ngn2 protein; and
viii) NeuroD1 protein or a nucleic acid encoding the same.

For example, the composition may include:
i) Gsta4 protein or a nucleic acid encoding the same;
ii) Hb9 protein or a nucleic acid encoding the same;
iii) Lhx3 protein or a nucleic acid encoding the same;
iv) Isl1 protein or a nucleic acid encoding the same;
v) Ascl1 protein or a nucleic acid encoding the same;
vi) Brn2 protein or a nucleic acid encoding the same;
vii) MytlL protein or a nucleic acid encoding the Ngn2 protein; and
viii) NeuroD1 protein or a nucleic acid encoding the same.

For example, the composition may include:
i) Gsta4 protein or a nucleic acid encoding the same;
ii) Hb9 protein or a nucleic acid encoding the same;
iii) Ngn2 protein or a nucleic acid encoding the same;
iv) Isl1 protein or a nucleic acid encoding the same;
v) Ascl1 protein or a nucleic acid encoding the same;
vi) Brn2 protein or a nucleic acid encoding the same;
vii) MytlL protein or a nucleic acid encoding the Ngn2 protein; and
viii) NeuroD1 protein or a nucleic acid encoding the same.

For example, the composition may include:
i) Gsta4 protein or a nucleic acid encoding the same;
ii) Lhx3 protein or a nucleic acid encoding the same;
iii) Ngn2 protein or a nucleic acid encoding the same;
iv) Isl1 protein or a nucleic acid encoding the same;
v) Ascl1 protein or a nucleic acid encoding the same;
vi) Brn2 protein or a nucleic acid encoding the same;
vii) MytlL protein or a nucleic acid encoding the Ngn2 protein; and
viii) NeuroD1 protein or a nucleic acid encoding the same.

### Composition (9): containing Gsta4 + eight conventional converting factors

The composition of the present application may include:
i) Gsta4 protein or a nucleic acid encoding the same; and
ii) Ascl1, Brn2, Myt1L, Hb9, Isl1, Lhx3, Ngn2 and NeuroD1 proteins; or nucleic acids encoding the same.

Here, the composition may take the form of a protein, a nucleic acid, or a mixture of a protein and a nucleic acid, but is not limited thereto. In this case, the nucleic acid may be DNA, RNA, or a mixture of DNA and RNA.

In one example, in the composition, all the factors may be included in the form of proteins.

In another example, in the composition, all the factors may be included in the form of nucleic acids.

In a further example, in the composition, some of the factors may be included in the form of proteins and the other factors are included in the form of nucleic acids.

The nucleic acid form may be a vector form. The vector may be composed of a single vector or composed of two or more vectors.

In addition, the composition may include a viral capsid.

The converting factor may be delivered as being contained in the viral capsid.

For example, the pharmaceutical composition of the present application may include a viral capsid containing a Gsta4 single gene.

For example, the composition may include: a viral capsid containing Gsta4; and
a viral capsid containing one or more selected from among Ascl1, Brn2, Myt1L, Hb9, Isl1, Lhx3, Ngn2, and NeuroD1.

### Vector for expression of converting factor

For example, the composition may include a converting factor. In this case, the vector may include a nucleic acid encoding Gsta4 protein. Alternatively, in addition to the nucleic acid encoding Gsta4 protein, a nucleic acid encoding a conventional converting factor may be further included.

The vector has a function of effectively expressing a desired converting factor in a target cell, and may further include other necessary components such as a promoter and an enhancer for this purpose.

### Essential component of vector - nucleic acid encoding Gsta4 protein

The vector essentially includes a nucleic acid encoding Gsta4 protein.

The nucleic acid may be a full-length sequence or a partial sequence of the Gsta4 gene.

For example, the partial sequence may be an exon sequence of Gsta4. In this case, the exon sequence may be a CDS sequence not containing introns compared to the Gsta4 gene.

For example, the vector may include a DNA sequence represented by SEQ ID NO: 2 or SEQ ID NO: 12.

### Optional component of vector - nucleic acid encoding conventional converting factor

Additionally, the vector may optionally include a nucleic acid encoding a conventional converting vector in addition to a nucleic acid encoding Gsta4 protein. The Gsta4 protein and the nucleic acid encoding a conventional converting factor may becontained in one vector, or two or more vectors.

In one embodiment, the vector may include nucleic acids encoding the converting factors selected from combination examples (1) to (8) of Gsta4 and conventional converting factors.

For example, the vector may include:
i) a sequence selected from SEQ ID NO: 2 and SEQ ID NO: 12; and
ii) a sequence selected from SEQ ID NOs: 14, 16, 18, 20, 22, 24, 26, and 28.

For example, the vector may include:
i) a sequence selected from SEQ ID NO: 2 and SEQ ID NO: 12; and
ii) two sequences selected from SEQ ID NOs: 14, 16, 18, 20, 22, 24, 26, and 28.

For example, the vector may include:
i) a sequence selected from SEQ ID NO: 2 and SEQ ID NO: 12; and
ii) three sequences selected from SEQ ID NOs: 14, 16, 18, 20, 22, 24, 26, and 28.

For example, the vector may include:
i) a sequence selected from SEQ ID NO: 2 and SEQ ID NO: 12; and
ii) four sequences selected from SEQ ID NOs: 14, 16, 18, 20, 22, 24, 26, and 28.

For example, the vector may include:
i) a sequence selected from SEQ ID NO: 2 and SEQ ID NO: 12; and
ii) five sequences selected from SEQ ID NOs: 14, 16, 18, 20, 22, 24, 26, and 28.

For example, the vector may include:
i) a sequence selected from SEQ ID NO: 2 and SEQ ID NO: 12; and
ii) six sequences selected from SEQ ID NOs: 14, 16, 18, 20, 22, 24, 26, and 28.

For example, the vector may include:
i) a sequence selected from SEQ ID NO: 2 and SEQ ID NO: 12; and
ii) seven sequences selected from SEQ ID NOs: 14, 16, 18, 20, 22, 24, 26, and 28.

For example, the vector may include:
i) a sequence selected from SEQ ID NO: 2 and SEQ ID NO: 12; and
ii) sequences of SEQ ID NOs: 14, 16, 18, 20, 22, 24, 26, and 28.

### Additional component of vector(1)

The vector may include, in addition to the essential and optional components of the target protein, additional components required for the expression of converting factors in a cell.

For example, the additional components may include expression control elements, selection elements, and the like.

The expression control elements include a promoter, an enhancer, a polyadenylation signal, a Kozak consensus sequence, an inverted terminal repeat (ITR), a long terminal repeat (LTR), a terminator, an internal ribosome entry site (IRES), 2A self-cleaving peptides, and the like.

For example, the promoter may be an SV40 early promoter, a mouse mammary tumor virus long terminal repeat (LTR) promoter, an adenovirus major late (Ad MLP) promoter, a herpes simplex virus (HSV) promoter, a cytomegalovirus (CMV) promoter, a rouse sarcoma virus (RSV) promoter, a U6 promoter, or the like.

For example, the 2A self-cleaving peptides may be T2A, P2A, E2A, F2A, and the like. The vector for expression of converting factors may contain one or more 2A self-cleaving peptides. In this case, the 2A self-cleaving peptide generates several proteins from the same transcript. Therefore, the 2A self-cleaving peptide can be placed between two or more different proteins intended to be expressed in the vector.

The selection element may be a fluorescent protein gene, a tag, a reporter gene, an antibiotic resistance gene, or the like.

For example, the fluorescent protein gene may be GFP gene, YFP gene, RFP gene, or mCherry gene.

For example, the tags may be a histidine (His) tag, a V5 tag, a FLAG tag, an influenza hemagglutinin (HA) tag, a Myc tag, a VSV-G tag, and a thioredoxin (Trx) tag.

For example, the reporter gene may be glutathione-S-transperase (GST), horseradish peroxidase (HRP), chloramphenicol acetyltransferase (CAT) beta-galactosidase, beta-glucuronidase, or the like.

For example, the antibiotic resistance gene may be a hygromycin resistant gene, a neomycin resistant gene, a kanamycin resistant gene, a blasticidin resistant gene, a zeocin resistant gene, or the like.

The vector of the present application includes essential components, optional components, and additional components and can be used to effectively express a desired converting factor.

In one embodiment, when the vector of the present application includes a nucleic acid encoding Gsta4 protein, the vector may have the sequence of SEQ ID NO: 36.

In one embodiment, when the expression vector includes a nucleic acid encoding Gsta4 protein and a nucleic acid encoding Mytll protein, the vector may have the sequence of SEQ ID NO: 5.

In another embodiment, the expression vector includes a nucleic acid encoding Gsta4 protein, a nucleic acid encoding MNX1 protein, and a nucleic acid encoding LHX3 protein, the vector may have the sequence of SEQ ID NO: 29.

### Additional component of vector (2) (for knock-in)

In another embodiment, the expression vector may further include an additional component (2) for knock-in of the converting factor into a target genome.

The term "knock-in" means inserting a converting factor into the genome of a target cell. In this case, since the expression of the inserted converting factor is maintained in the cell, the efficiency of conversion of the target cell (for example, somatic cell) into a motor neuron may be further increased.

In one embodiment, the converting factor may be knocked in a safe harbor region within the target cell genome.

The safe harbor refers to a genetic locus at which the converting factor is continuously expressed without causing side effects even when an exogenous converting factor is inserted into a specific part of the genome of a subject.

For example, the safe harbor may be a locus region encoding AAVS1, ROSA26, and the like in the genomic sequence of the target cell.

The knock-in of the converting factor may be performed by a method known in the art.

In one embodiment, the method may use a transposable element (TE) system, a CRISPR/Cas system, or the like.

The TE system uses a DNA fragment capable of moving around in a genome.

For example, the TE system may include a piggyBac TE, a Sleeping Beauty TE, and the like.

The CRISPR/Cas system may be composed of a Cas protein, a guide RNA, and a donor. In this case, the donor may include (i) a converting factor to be expressed, and (ii) a sequence having homology with some sequences of the safe harbor gene in the target cell genome. In this case, the sequence having homology to some sequences of the safe harbor gene of the (ii) may function as homology arms at respective ends of the donor.

For example, the Cas protein and the guide RNA form a complex to cause double strand break (DSB) cleavage in the safe harbor gene sequence in the target cell genome, and at this time, at the cleavage site, a converting factor (donor) sequence having homologous arms at respective ends may be inserted via a homology-directed repair pathway (HDR). In this case, the two homology arms are a sequence having homology with some sequences upstream of the cleavage site and a sequence having homology with some sequences downstream of the cleavage site.

### Vector types

In one example, the vector may be a viral vector.

The viral vector may be selected from among a retrovirus, a lentivirus, an adenovirus, an adeno-associated virus (AAV), a vaccinia virus, a poxvirus, a human immunodeficiency virus (HIV), a murine leukemia virus (MLV), an Avian sarcoma/leukosis (ASLV), a SNV (Spleen necrosis virus), a Rous sarcoma virus (RSV), a Mouse mammary tumor virus (MMTV), a Herpes simplex virus, an episomal, a herpes simplex virus, and the like. Preferably, the viral vector may be an adenovirus or an adeno-associated virus (AAV).

In another example, the vector may be a non-viral vector.

For example, the non-viral vector may be a plasmid, phage, a naked DNA, a DNA-lipid complex, a DNA-polymer complex, mRNA, and the like.

### 4. Direct cell-conversion method

### Overview of direct cell-conversion method

In another aspect of the present application, there is provided a method for direct cell-conversion from a somatic cell into a motor neuron using the composition for expression of the converting factor described above.

Accordingly, the present application discloses a direct cell-conversion method using a composition containing Gsta4.

By using the composition, target cells can be directly converted into motor neurons without involving a dedifferentiation step. The direct cell-conversion may be performed by appropriately selecting an existing method known to a person skilled in the art, and is not particularly limited if it is possible to achieve the above objectives by the method.

In one embodiment, the direct cell-conversion method includes a step of introducing a composition selected from compositions (1) to (9) into a target cell.

The composition essentially contains Gsta4, and
the direct cell-conversion method does not show the effect of dedifferentiating the target cell into an induced pluripotent stem cell (iPSC).

In an optional embodiment, the direct cell-conversion method of the present application may include a step of introducing a vector containing a nucleic acid encoding sta4 protein into a target cell.

In one embodiment, the direct cell-conversion method of the present application may include a step of introducing a vector including:
Gsta4 protein or a nucleic acid encoding the same; and
one or more proteins selected from Ascl1, Brn2, Myt1L, Hb9, Isl1, Lhx3, Ngn2 and NeuroD1, or one or more nucleic acids encoding the respective selected proteins.

In one specific embodiment, the direct cell-conversion method of the present application may include a step of introducing a vector containing a nucleic acid encoding Gsta4 protein, a nucleic acid encoding Lhx3 protein, and a nucleic acid encoding Hb9 protein into a target cell.

### Target cell (starting cell) for direct cell-conversion

The direct cell-conversion that the present application intends to achieve is introducing the factor of the present application into a differentiated cell to directly convert the differentiated cell to the desired cell.

The differentiated cell, which is the target cell used in the direct cell-conversion method of the present application, may be a somatic cell. The somatic cells refer to all the cells in the body other than germ cells.

For example, the somatic cell may be selected from among a fibroblast, an epithelial cell, an endothelial cell, a muscle cell, a nerve cell, a cell derived from hair, a cell derived from hair root, a hair follicle cell, an oral epithelial cell, a somatic cell extracted from urine, a gastric mucosal cell, a goblet cell , a gastrin cell/G cell, a B cell, a pericyte, an astrocyte, a blood cell, and an oligodendrocyte progenitor cell.

The somatic cells may be derived or isolated from mammals such as humans, dogs, cats, horses, sheep, rabbits, pigs, mice, and camels.

For example, the direct cell-conversion method of the present application includes:
introducing a composition for expression of a converting factor for the direct cell-conversion into a somatic cell.

In this case, the somatic cell may be a somatic cell cultured for a certain period before the introduction of the composition.

The cell-conversion method may further include a process of culturing the somatic cell into which the composition has been introduced in a culture medium for neuronal differentiation.

Hereinafter, a culture medium for culturing somatic cells, a culture medium for differentiation of nerve cells, a differentiation period (i.e., a period for culturing somatic cells into which the composition has been introduced), and an introduction method, which are used in the cell-conversion method of the present application, will be described.

### Culture medium

The culture medium may be a culture medium for somatic cell culture or a culture medium for neuronal differentiation, depending on the purpose.

As the culture medium for somatic cell culture and the culture medium for neuronal differentiation, a culture medium known in the art may be used. In addition, the culture medium may be appropriately changed when used for a predetermined purpose. For example, to increase the efficiency of direct cell-conversion, which is the purpose of the present application, carbon sources, nitrogen sources, trace elements, growth factors, etc. known in the art may be appropriately adjusted.

For example, the culture medium may be DMEM, MEM, RPMI-1640, Ham's F-10, or Ham's F-12.

### Cell confluence, culture period, and subculture

When culturing somatic cells, the confluence of the cells can be adjusted to facilitate experiments.

In this case, the confluence of the somatic cells can be appropriately adjusted according to the cell state before or after the composition is introduced into the somatic cells.

Somatic cells may be cultured until the confluence of the cells reaches a range of about 60% to 100%. Preferably, somatic cells may be cultured until the confluence reaches a range of about 80% to 100%.

In addition, the somatic cell culture may be performed for about 12 to 60 hours to reach an appropriate confluence for easy experimentation, but the period is not limited thereto.

For the somatic cell culture, the somatic cells may be subcultured 1 to 3 times for proper confluence, but is not limited thereto.

### Order of introduction of each component of the composition for the expression of converting factors

In the direct cell-conversion method, the order of introducing a composition selected from compositions (1) to (9) into a target cell is not particularly limited. For example, when the composition includes two or more converting factors, the converting factors may be introduced simultaneously or sequentially into the target cell.

For example, in the direct cell-conversion method of the present application, an expression vector containing a nucleic acid encoding Gsta4 protein, an expression vector containing a nucleic acid encoding Lhx3 protein, and an expression vector containing a nucleic acid encoding Hb9 protein may be introduced simultaneously or sequentially.

### Method for introducing a converting factor expression composition into a target cell

In the direct cell-conversion method, the introduction of the composition for expression of a converting factor for direct cell-conversion into a target cell may be performed by a person skilled in the art by appropriately selecting a known technique.

For example, to introduce the composition into a cell, electroporation, gene gun, sonoporation, magnetofection, microinjection, transient cell compression or squeezing, cationic liposomal method, lithium acetate-DMSO, lipid-mediated transfection, phosphoric acid Calcium precipitation, lipofection, polyethyleneimine (PEI)-mediated transfection, or DEAE-dextran-mediated transfection may be used, but the method is not limited thereto.

### Characteristics of somatic cells introduced with the composition for expression of converting factors

Somatic cells introduced with the converting factor expression composition of the present application are in a state in which the expression of converting factors and neuronal markers increases as the culture time passes.

The neuronal marker may be NEUN, ChAT, Map2, Hb9, Synapsin, Tuj1, IsL1, PAX6, Olig2, Nkx2.2, or SMI-32.

For example, in the somatic cells introduced with the composition for expression of the converting factors of the present application, the expression of one or more neuronal markers selected from among NEUN, ChAT, Map2, Hb9, and Synapsin may be increased on the third day of culture compared to the first day of culture.

### Conversion period

When converting factors are introduced into somatic cells by the direct cell-conversion method described above, the somatic cells are converted into motor neurons after an appropriate period. Until the somatic cells are converted into motor neurons, the conversion environment can be maintained by changing the composition of the culture medium. The appropriate time may be determined according to the target cell.

For example, fibroblasts introduced with the converting factor expressing composition can be converted into motor neurons within about 7 to 30 days after the introduction.

For another example, astrocytes introduced with the converting factor expressing composition can be converted into motor neurons within about 7 to 30 days after the introduction.

### Result of direct cell-conversion method

Using the above-described direct cell-conversion of the present application, motor neurons can be obtained by direct cell conversion of somatic cells. Motor neurons are nerve cells that transmit motor stimuli from the brain and spinal cord to muscles or glands.

In particular, the motor neurons generated by introduction of the direct cell-conversion factor into somatic cells are characterized in that the converting factors are over-expressed in the cells. Therefore, the induced motor neurons may have a characteristic in that neuronal markers are also over-expressed due to the over-expression of the converting factors.

The direct cell-conversion factor may be selected from Gsta4, Ascl1 (Achaete-scute homolog 1), Brn2 (POU Class 3 Homeobox 2), MytlL (Myelin Transcription Factor 1 Like), Hb9 (Homeobox HB9), ISL1 (ISL LIM homeobox 1), Lhx3 (LIM homeobox 3), Ngn2 (neurogenin 2), and NeuroD1 (neuronal differentiation 1) but is not limited thereto.

For example, Gsta4 is over-expressed in motor neurons obtained by the direct cell-conversion.

For example, one or more direct cell-conversion factors selected from the following are over-expressed in the motor neurons obtained by direct cell-conversion: Gsta4; and one or more converting factors selected from Ascl1 (Achaete-scute homolog 1), Brn2 (POU Class 3 Homeobox 2), MytlL (Myelin Transcription Factor 1 Like), Hb9 (Homeobox HB9), ISL1 (ISL LIM homeobox 1), Lhx3 (LIM homeobox 3), Ngn2 (neurogenin 2), and NeuroD1 (neuronal differentiation 1).

In addition, over-expression of these converting factors causes an increase in expression of neuronal marker proteins in motor neurons. The neuronal markers may include NEUN, ChAT, Map2, Synapsin, HB9, Tuj1, IsL1, Notch1, HES1, HES3, E-cadherin, occludin, PAX6, N-cadherin, SOX2, etc., but are not limited thereto.

For example, in the motor neurons obtained by direct cell-conversion, one or more neuronal markers selected from ChAT, Map2, Hb9, and Synapsin may be over-expressed.

In another example, in the motor neurons obtained by the direct cell-conversion, one or more of the induced Tuj1, IsL1, and NEUN may be over-expressed.

### Confirmation of results of direct cell-conversion

The direct cell-conversion method of the present application may optionally further include a step of confirming conversion into motor neurons.

The confirmation process can be performed using a neuronal marker known in the art, and can be performed by various molecular biology techniques known in the art. For example, the molecular biology techniques include immunofluorescence staining, western blotting, polymerase chain reaction (PCR), reverse transcription polymerase chain reaction (RT-PCR), enzyme-linked immunosorbent assay (ELISA), and the like but are not limited thereto.

### Characteristics (1) of direct cell-conversion method of present application

The direct cell-conversion method of the present application has a characteristic in that induced pluripotent stem cells (iPSCs) are not generated. That is, it is characterized in that somatic cells can be directly converted into motor neurons without going through the iPSC stage involving somatic cell dedifferentiation.

For example, when fibroblasts or astrocytes are treated with only Gsta4 alone or treated with Gsta4 and a conventional converting factor together, the fibroblasts or astrocytes can be directly converted into motor neurons.

### Characteristics (2) of direct cell-conversion method of present application

In addition, since the method of the present application does not involve the induced pluripotent stem cell stage as described above, there is an advantage in that teratoma of the target cells are not likely to be formed. That is, the method of the present application may be a method that significantly lowers the probability of teratoma formation, which is a problem of conventional stem cell use. These advantages suggest that the method of the present application can be more usefully used for the treatment of nerve system diseases.

### Characteristics (3) of direct cell-conversion method of present application

The direct cell-conversion method disclosed in the present application greatly contributes to improvement of conversion efficiency, which is a problem of conventional converting factors. When fibroblasts or astrocytes are treated with Gsta4 and conventional converting factors together, the conversion efficiency of direct cell-conversion into motor neurons can be significantly improved compared to the case where fibroblasts or astrocytes are treated with only conventional converting factors.

### 5. Pharmaceutical composition for direct cell-conversion containing Gsta4

### Overview of pharmaceutical composition for direct cell-conversion containing Gsta4

In another aspect, the present application discloses a composition selected from the compositions (1) to (9) as a pharmaceutical composition for the treatment of nerve system diseases. Hereinafter, components that may be included in the pharmaceutical composition and specific indications for therapeutic use of the pharmaceutical composition will be described.

### Active ingredient of pharmaceutical Composition

The active ingredients of the pharmaceutical composition of the present application may be at least one of the following:
(1) the converting factor of the present application;
(2) a somatic cell containing the converting factor of the present application; and
(3) a motor neuron induced with the use of the converting factor of the present application.

The converting factor is Gsta4 alone. Alternatively, the converting factor is a combination of Gsta4 and conventional differentiation factors. The conventional converting factors may be one or more proteins selected from Ascl1, Brn2, Myt1L, Hb9, Isl1, Lhx3, Ngn2, and NeuroD1.

The (1) converting factor may be in the form of a protein or a nucleic acid.

For example, the pharmaceutical composition for preventing or treating a nerve system disease, of the present application, may include Gsta4 protein or a nucleic acid encoding the same.

Alternatively, the pharmaceutical composition for preventing or treating a nerve system disease, of the present application, may include: Gsta4 protein or a nucleic acid encoding the same; Hb9 protein or a nucleic acid encoding the same; and Lhx3 protein or a nucleic acid encoding the same.

Further alternatively, the pharmaceutical composition for preventing or treating a nerve system disease, of the present application, may include: Gsta4 protein or a nucleic acid encoding the same; Hb9 protein or a nucleic acid encoding the same; Lhx3 protein or a nucleic acid encoding the same; and Ngn2 protein or a nucleic acid encoding the same.

The (1) converting factor may be delivered as being contained in a viral capsid. For example, the pharmaceutical composition of the present application may include a viral capsid containing a Gsta4 single gene. Further alternatively, the composition may include: a viral capsid containing Gsta4; a viral capsid containing Hb9; and a viral capsid containing Lhx3. When the viral capsid containing the converting factor of the present application is included, the composition can be used as a kind of gene therapy.

In addition, the (2) somatic cell containing the converting factor may be: i) a somatic cell into which a vector containing a nucleic acid encoding the converting factor has been introduced; or ii) a somatic cell into which a converting factor protein has been introduced. When the somatic cells containing the converting factor of the present application are included as an active ingredient, the composition can be used as a type of cell therapy. The cell therapy refers to a therapeutic agent that uses autologous, allogenic, or xenogenic cells as an active ingredient to restore tissue function.

For example, the pharmaceutical composition for preventing or treating a nerve system disease, of the present application, may include a somatic cell into which a vector containing a nucleic acid encoding Gsta4 protein has been introduced. The pharmaceutical composition for preventing or treating a nerve system disease, of the present application, may include a somatic cell into which Gsta4 protein has been introduced.

Alternatively, the pharmaceutical composition for preventing or treating a nerve system disease, of the present application, may include: a somatic cell containing a vector containing a nucleic acid encoding Gsta4 protein; a nucleic acid encoding Hb9 protein; and a nucleic acid encoding Lhx3 protein. For example, the pharmaceutical composition for preventing or treating a nerve system disease, of the present application, may include: a somatic cell containing Gsta4 Protein; Hb9 protein; and Lhx3 protein.

In addition, the motor neurons derived using the (3) converting factor are motor neurons produced by directly converting somatic cells using the [4. Direct cell-conversion method]. When the induced motor neurons are contained as an active ingredient, the composition can be used as another type of cell therapy.

### Pharmaceutically acceptable extra components

The pharmaceutical composition may further include pharmaceutically acceptable extra components in addition to the active ingredient. The pharmaceutically acceptable extra components may be carriers, excipients, diluents, preservatives, etc., but are not limited thereto.

For example, the carriers, excipients, and diluents are lactose, dextrose, sucrose, sorbitol, mannitol, xylitol, erythritol, maltitol, starch, gum acacia, alginates, gelatin, calcium phosphate, calcium silicate, cellulose, methyl cellulose, distilled water, physiological saline, glycerol, ethanol, human serum albumin (HSA), and the like.

For example, the preservative may be benzoic acid, sodium benzoate, sorbic acid, paraoxybenzoic acid, chlorobutanol, and the like.

When formulating the pharmaceutical composition, fillers, extenders, binders, wetting agents, and the like may be further included.

### Formulation of pharmaceutical composition

The pharmaceutical composition may be formulated for oral or parenteral use.

For example, when formulated for oral use, the pharmaceutical composition may be prepared in solid form, liquid form, capsule form, semi-solid form, and the like.

When formulated for parenteral use, the pharmaceutical composition may be prepared as an injection, an aerosol, and the like. Preferably, it may be formulated as an injection.

### Target disease of pharmaceutical composition

The pharmaceutical composition may be applied to nervous system diseases. In particular, the pharmaceutical composition may be used for the treatment or prevention of nerve system diseases. In the present application, the nervous system disease may be damage to neurons or a disease caused thereby.

For example, the nervous system disease may be one or more selected from Spinal cord injury, Parkinson's disease, stroke, Huntington's disease, Lou Gehrig's disease, Ataxia telangiectasia, amyotrophic lateral sclerosis, motor neuron damage, traumatic peripheral nerve damage, ischemic brain injury, neonatal hypoxic ischemia Brain damage, cerebral palsy, peripheral palsy, central paralysis, quadriplegia, biparesis, epilepsy, neuronal development disorder, neuralgia, intractable epilepsy, Alzheimer's disease, congenital metabolic nervous system disease, traumatic brain injury, motor nerve cell damage, or a disease caused by the motor nerve cell damage, but is not limited thereto. Preferably, the nervous system disease may be selected from among spinal cord injury, Parkinson's disease, Alzheimer's disease, amyotrophic lateral sclerosis, peripheral paralysis, central paralysis, quadriplegia, and limb palsy.

### 6. Nervous system disease treatment method

### Overview of treatment method for nervous system disease

A nervous system disease treatment method using the [5. Pharmaceutical composition containing Gsta4 for direct cell-conversion] described above will be described below.

As described above, the pharmaceutical composition includes:
Gsta4 or
a combination of Gsta4 and at least one selected from Ascl1, Brn2, Myt1L, Hb9, Isl1, Lhx3, Ngn2, and NeuroD1.

The nervous system disease treatment method of the present application is (i) treating the subject (patient) with the composition as it is, (ii) treating the subject with the somatic cells introduced with the composition, or (iii) treating the subject with motor neurons derived ex vivo. The method (i) may have an effect of generating motor neurons induced in the body of the subject, that is, in vivo.

### Treatment method (1)

The nervous system disease treatment method may use a method in which motor neurons induced in vivo by injecting the "pharmaceutical composition" of the present application into a subject in need of treatment or prevention of a nervous system disease.

For example, the nervous system disease treatment method of the present application may include:
administering to a subject a composition including Gsta4 protein or a nucleic acid encoding the same.

Alternatively, the nervous system disease treatment method may include administering to a subject a composition including:
i) Gsta4 (Glutathione S-Transferase Alpha 4) protein or a nucleic acid encoding the same; and
at least one protein selected from Ascl1, Brn2, Myt1L, Hb9, Isl1, Lhx3, Ngn2, and NeuroD1 or one or more nucleic acids encoding the selected proteins.

### Treatment method (2)

The nervous system disease treatment method is characterized by injecting "somatic cells" introduced with the pharmaceutical composition of the present application, into a subject.

For example, the nervous system disease treatment method of the present application may include:
administering somatic cells introduced with Gsta4 protein or a nucleic acid encoding the same, to a subject.

Alternatively, the nervous system disease treatment method may include administering, to a subject, somatic cells introduced with a composition including:
i) Gsta4 protein or a nucleic acid encoding the same; and
ii) one or more proteins selected from Ascl1, Brn2, Myt1L, Hb9, Isl1, Lhx3, Ngn2, and NeuroD1, or one or more nucleic acids encoding the selected proteins.

As described above, when somatic cells introduced with the composition of the present application are injected into a subject, the somatic cells are directly converted into motor neurons in the body of the subject due to the converting factors present in the somatic cells. As a result, the motor neurons proliferate in the subject, thereby treating or preventing the nerve system disease.

### Treatment method (3)

The nervous system disease treatment method may use a method in which the composition of the present application is introduced into isolated somatic cells to obtain motor neurons induced ex vivo, and the "motor neurons" are injected into a subject in need. That is, it is a method of applying the induced motor neurons of the present application to a subject as a direct active ingredient of a therapeutic agent.

For example, the nervous system disease treatment method of the present application may include:
administering, to a subject, an induced motor neuron (iMN) produced by introducing a composition including Gsta4 protein or a nucleic acid encoding the same into a somatic cell.

Alternatively, the nervous system disease treatment method may include administering,
to a subject,
induced motor neurons produced by introducing, into somatic cells, a composition including:
   i) Gsta4 protein or a nucleic acid encoding the same; and
   ii) one or more proteins selected from Ascl1, Brn2, Myt1L, Hb9, Isl1, Lhx3, Ngn2, and NeuroD1, or one or more nucleic acids encoding the selected proteins.

### Administration target

The subject of the nervous system disease treatment method may be a subject whose motor neuron function is impaired compared to a healthy person or a subject whose disease occurs due to a decrease in the number of motor neurons, but is not limited thereto.

The subject in need of treatment or prevention of a nervous system disease may be a mammal. For example, the mammal may be a human, dog, horse, cat, mouse, rabbit, sheep, monkey, and the like.

The injection method, dosage, concentration of the pharmaceutical composition, and injection cycle, which will be described below, will be determined depending on the age, body weight, general health condition, diet, degree of nerve system disease, intake of other drugs, treatment period, etc.

### Administration method

In the nerve system disease treatment method, the administration may be performed through various routes.

The administration may be oral administration or parenteral administration. The parenteral administration may be injection.

The site of injection administration may be, but is not limited to, muscle, intradermal, subcutaneous, venous, abdominal, arterial, mucosal, spinal cord, bone marrow, intrathecal, transdermal, etc.

For example, when the injection site is the spinal cord, it may be specifically L1 (lumbar 1), L2 (lumbar 2), L3 (lumbar 3), L4 (lumbar 4), L5 (lumbar 5), T1 (thoracic 1), T2 (thoracic 2), T3 (thoracic 3), T4 (thoracic 4), T5 (thoracic 5), T6 (thoracic 6), T7 (thoracic 7), C1 (cervical 1), C2 (cervical 2), C3 (cervical 3) , C4 (cervical 4), or C5 (cervical 5).

Alternatively, it can be administered to the site where the nervous system disease has occurred. In some embodiments, it may be administered directly to the brain affected by Parkinson's disease.

### Concentration of pharmaceutical composition

The concentration of the pharmaceutical composition may be as follows based on the total composition. In this case, the pharmaceutical composition may be a form containing a vector, a form containing a virus purified from a vector, a cell form containing a vector, a protein form, a cell form containing a protein, etc., but is not limited thereto.

For example, when a viral vector containing the pharmaceutical composition of the present application is purified as a virus, the virus may be included at the following concentrations based on the total amount of the composition.

For example, the concentration may be in the range of from 1×10⁵ vg (viral genome)/mL to 1×10²⁰ vg (viral genome)/mL with respect to the total amount of the pharmaceutical composition.

For example, the concentration of the virus may be 1×10⁵ GC (genome copies) /mL to 1 × 10²⁰ GC (genome copies) /mL based on the total pharmaceutical composition.

Alternatively, the concentration of the virus is 1×10⁵ VP (viral particles)/mL to 1×10²⁰ VP (viral particles)/mL based on the total pharmaceutical composition.

### Administration dosage

The administration dose of the pharmaceutical composition may be determined depending on the administration method, subject, and the like.

For example, the administration dose may be 1 uL to 20 mL for a single administration, but is not limited thereto.

In a specific example, 1 uL/kg to 20 uL/kg may be administered at a time for spinal cord administration. When administered intravenously, 0.5 mL/kg to 10 mL/kg may be administered at a time.

### Administration cycle

The frequency of administration of the pharmaceutical composition to a subject may be determined depending on the type of disease, severity, etc. of the subject.

The frequency of administration may be once or several times a day, or several times over a long period of time. It may be administered at intervals of a certain period.

The intervals may range from 1 day to 60 days. For the administration, continuous administration or non-continuous administration may be performed.

### Examination of treatment effect

After applying the nervous system disease treatment method to an administration target, the effect of alleviating, improving, or treating a nervous system disease can be examined in various ways.

For example, it can be examined through scar reduction, changes in neuronal marker expression, BBB score, action potential, spontaneous response, and the like.

For example, scars can be reduced by about 2 to 50 times by the nervous system disease treatment method.

Alternatively, the expression of neuronal markers can be increased by about 2 to 50 times by the nervous system disease treatment method.

Further alternatively, the BBB score can be increased by about 2 to 50 times by the nervous system disease treatment method.

Yet further alternatively, when using the nervous system disease treatment method, an action potential or a spontaneous response may appear in a pattern similar to that of a healthy person.

### 7. Uses of induced motor neurons

### Overview of uses of induced motor neurons

The present application discloses various uses of motor neurons obtained by the direct cell-conversion method. Hereinafter, as exemplary uses of the motor neurons, drug screening, biological materials, and the like will be described.

### Use (1) of motor neurons - drug screening

For example, as a use of the induced motor neurons of the present application, drug screening is disclosed. In particular, the motor neurons can be used for screening of drugs for the prevention or treatment of nervous system diseases.

In a manner of checking response of motor neurons before and after injection of candidate drugs into the induced motor neurons, the motor neurons can be usefully used for the screening of drugs for the prevention or treatment of a nervous system disease.

### Use (2) of motor neurons - artificial organ

For example, an artificial organ including the induced motor neurons of the present application is disclosed. An organoid is a kind of artificial organ that mimics a real organ by inducing stem cells into a specific organ in vitro. Since the present application involves the induced pluripotent stem cell stage, it can be used for artificial organs with fewer side effects than organoids using stem cells.

The motor neurons of the present application can be applied to 3D bioprinting. 3D bioprinting is the process of making artificial organs such as nerves, corneas, livers, skins, and blood vessels by stacking a bioink using living cells, layer by layer, like 3D printing. Therefore, artificial organs for treating nervous system diseases or injuries can be manufactured by 3D bioprinting of the induced motor neurons of the present application.

### [Possible embodiment of the invention]

### Embodiment 1: Gsta4 alone

A composition for direct cell-conversion of somatic cells into motor neurons, the composition including:
glutathione S-transferase A4 (Gsta4) protein or a nucleic acid encoding the same,
in which the motor neurons are motor neurons derived (differentiated) from the somatic cells.

(A composition characterized in that somatic cells are directly converted to become induced motor neurons.)

### Embodiment 2: Gsta4 + one conventional factor

In Embodiment 1,
the composition further includes:
one protein selected from Ascl1 (Achaete-scute homolog 1), Brn2 (POU Class 3 Homeobox 2), MytlL (Myelin Transcription Factor 1 Like), Hb9 (Homeobox HB9), ISL1 (ISL LIM homeobox 1), Lhx3 (LIM homeobox 3), Ngn2 (neurogenin 2), and NeuroD1 (neuronal differentiation 1), or a nucleic acid encoding the selected protein.

### Embodiment 3: Gsta4 + two conventional factors

In Embodiment 1,
the composition further includes:
two proteins selected from among Ascl1 (Achaete-scute homolog 1), Brn2 (POU Class 3 Homeobox 2), MytlL (Myelin Transcription Factor 1 Like), Hb9 (Homeobox HB9), ISL1 (ISL LIM homeobox 1), Lhx3 (LIM homeobox 3), Ngn2 (neurogenin 2), and NeuroD1 (neuronal differentiation 1), or nucleic acids encoding the selected proteins.

### Embodiment 4: Gsta4 + three conventional factors

In Embodiment 1,
the composition further includes:
three proteins selected from among Ascl1 (Achaete-scute homolog 1), Brn2 (POU Class 3 Homeobox 2), MytlL (Myelin Transcription Factor 1 Like), Hb9 (Homeobox HB9), ISL1 (ISL LIM homeobox 1), Lhx3 (LIM homeobox 3), Ngn2 (neurogenin 2), and NeuroD1 (neuronal differentiation 1), or nucleic acids encoding the selected proteins.

### Embodiment 5: Gsta4 + four conventional factors

In Embodiment 1,
the composition further includes:
four proteins selected from among Ascl1 (Achaete-scute homolog 1), Brn2 (POU Class 3 Homeobox 2), MytlL (Myelin Transcription Factor 1 Like), Hb9 (Homeobox HB9), ISL1 (ISL LIM homeobox 1), Lhx3 (LIM homeobox 3), Ngn2 (neurogenin 2), and NeuroD1 (neuronal differentiation 1), or nucleic acids encoding the selected proteins.

### Embodiment 6: Gsta4 + five conventional factors

In Embodiment 1,
the composition further includes:
five proteins selected from among Ascl1 (Achaete-scute homolog 1), Brn2 (POU Class 3 Homeobox 2), MytlL (Myelin Transcription Factor 1 Like), Hb9 (Homeobox HB9), ISL1 (ISL LIM homeobox 1), Lhx3 (LIM homeobox 3), Ngn2 (neurogenin 2), and NeuroD1 (neuronal differentiation 1), or nucleic acids encoding the selected proteins.

### Embodiment 7: Gsta4 + six conventional factors

In Embodiment 1,
the composition further includes:
six proteins selected from among Ascl1 (Achaete-scute homolog 1), Brn2 (POU Class 3 Homeobox 2), MytlL (Myelin Transcription Factor 1 Like), Hb9 (Homeobox HB9), ISL1 (ISL LIM homeobox 1), Lhx3 (LIM homeobox 3), Ngn2 (neurogenin 2), and NeuroD1 (neuronal differentiation 1), or nucleic acids encoding the selected proteins.

### Embodiment 8: Gsta4 + seven conventional factors

In Embodiment 1,
the composition further includes:
seven proteins selected from among Ascl1 (Achaete-scute homolog 1), Brn2 (POU Class 3 Homeobox 2), MytlL (Myelin Transcription Factor 1 Like), Hb9 (Homeobox HB9), ISL1 (ISL LIM homeobox 1), Lhx3 (LIM homeobox 3), Ngn2 (neurogenin 2), and NeuroD1 (neuronal differentiation 1), or nucleic acids encoding the selected proteins.

### Embodiment 9: Gsta4 + eight conventional factors

In Embodiment 1,
the composition further includes:
eightproteins selected from among Ascl1 (Achaete-scute homolog 1), Brn2 (POU Class 3 Homeobox 2), MytlL (Myelin Transcription Factor 1 Like), Hb9 (Homeobox HB9), ISL1 (ISL LIM homeobox 1), Lhx3 (LIM homeobox 3), Ngn2 (neurogenin 2), and NeuroD1 (neuronal differentiation 1), or nucleic acids encoding the selected proteins.

### Embodiment 10: Combination of components

In Embodiment 2,
the composition includes:
Gsta4 protein or a nucleic acid encoding the same; and
Brn2 protein or a nucleic acid encoding the same, or Ascl1 protein or a nucleic acid encoding the same.

### Embodiment 11: Combination of components

In Embodiment 3,
the composition includes:
Gsta4 protein or a nucleic acid encoding the same;
Hb9 protein or a nucleic acid encoding the same; and
Lhx3 protein or a nucleic acid encoding the same.

### Embodiment 12: Combination of components

In Embodiment 4,
the composition includes:
Gsta4 protein or a nucleic acid encoding the same;
Brn2 protein or a nucleic acid encoding the same;
Hb9 protein or a nucleic acid encoding the same; and
NeuroD1 protein or a nucleic acid encoding the same.

### Embodiment 13: Combination of components

In Embodiment 5,
the composition includes:
Gsta4 protein or a nucleic acid encoding the same;
Brn2 protein or a nucleic acid encoding the same;
Hb9 protein or a nucleic acid encoding the same;
Ascl1 protein or a nucleic acid encoding the same; and
MytlL protein or a nucleic acid encoding the same.

### Embodiment 14: Combination of components

In Embodiment 6,
the composition includes:
Gsta4 protein or a nucleic acid encoding the same;
Brn2 protein or a nucleic acid encoding the same;
Ascl1 protein or a nucleic acid encoding the same;
Ngn2 protein or a nucleic acid encoding the same;
Isl1 protein or a nucleic acid encoding the same; and
Lhx3 protein or a nucleic acid encoding the same.

### Embodiment 15: Combination of components

In Embodiment 7,
the composition includes:
Gsta4 protein or a nucleic acid encoding the same;
Brn2 protein or a nucleic acid encoding the same;
Ascl1 protein or a nucleic acid encoding the same;
Ngn2 protein or a nucleic acid encoding the same;
Isl1 protein or a nucleic acid encoding the same;
Lhx3 protein or a nucleic acid encoding the same; and
MytlL protein or a nucleic acid encoding the same.

### Embodiment 16: Gsta4 sequence

In Embodiment 1,
the composition is characterized in that the Gsta4 protein is an amino acid sequence represented by SEQ ID NO: 1 or an amino acid sequence having 70% or more sequence identity thereto.

### Embodiment 17: Hb9 sequence

In Embodiment 2,
the composition is characterized in that the Hb9 protein is an amino acid sequence represented by SEQ ID NO: 14 or an amino acid sequence having 70% or more sequence identity thereto.

### Embodiment 18: Lhx3 sequence

In Embodiment 2,
the composition is characterized in that the Lhx3 protein is an amino acid sequence represented by SEQ ID NO: 15 or an amino acid sequence having 70% or more sequence identity thereto.

### Embodiment 19: Sequence restriction

In Embodiment 11,
the composition is characterized in that the Gsta4 protein is an amino acid sequence represented by SEQ ID NO: 1 or an amino acid sequence having 70% or more sequence identity thereto,
the Hb9 protein is an amino acid sequence represented by SEQ ID NO: 14 or an amino acid sequence having 70% or more sequence identity thereto, and
the composition is characterized in that the Lhx3 protein is an amino acid sequence represented by SEQ ID NO: 15 or an amino acid sequence having 70% or more sequence identity thereto.

### Embodiment 20: Vector 1

A vector for direct cell-conversion of somatic cells into motor neurons, the vector including:
i) Embodiment 1 or 16; and
ii) a promoter,

in which the i) is operably linked to the ii), and
the motor neurons are motor neurons induced(differentiated) from the somatic cells.

### Embodiment 21: Extra component of Vector 1

In Embodiment 20,
the vector further includes one embodiment selected from Embodiments 2 to 9.

### Embodiment 22: Vector 2

A vector for direct cell-conversion of somatic cells into motor neurons, the vector including:
i) Embodiment 3 or 11; and
ii) a promoter,

in which the i) is operably linked to the ii), and
the motor neurons are motor neurons induced(differentiated) from the somatic cells.

### Embodiment 23: Vector 3

A vector for direct cell-conversion of somatic cells into motor neurons, the vector including two or more vectors,
the two or more vectors include a first vector and a second vector,
the first vector includes:
   i) Embodiment 1 or 16; and
   ii) a promoter,
in which the i) is operably linked to the ii), and
the second vector includes:
   iii) one or more embodiments selected from Embodiments 2 to 9; and
   iv) a promoter,
in which the iii) is operably linked to the iv), and
in the vector of the iii), at least one selected from among Embodiments 2 to 9 is included in an individual vector, and
the motor neurons are motor neurons induced (differentiated) from the somatic cells.

### Embodiment 24: Extra components of vector

In any one embodiment of Embodiments 21 to 23,
the vector optionally includes one or more selected from among a promoter other than ii), an enhancer, a polyadenylation signal, a Kozak consensus sequence, an inverted terminal repeat(ITR), a long terminal repeat(LTR), a terminator, an internal ribosome entry site(IRES) , fluorescent protein gene, a glutathione-S-transferase(GST), a horseradish peroxidase(HRP), a chloramphenicol acetyltransferase (CAT) beta-galactosidase, a beta-glucuronidase, a luciferase, a histidine (His) tag, a V5 tag, a FLAG tag, an influenza hemagglutinin (HA) tag, a Myc tag, a 2A self-cleaving peptide, and an antibiotic resistance gene.

### Embodiment 25: Restriction of extra components of vector

In Embodiment 24,
the vector characterized in that the 2A self-cleaving peptide is selected from among T2A, P2A, E2A, and F2A.

### Embodiment 26: Restriction of extra components of vector

In any one embodiment of Embodiments 21 to 23,
the vector further includes one or more selected from among a donor, a piggyBac transfer factor, and a Sleeping Beauty transfer factor.

### Embodiment 27: Restriction of vector types

In any one embodiment of Embodiments 21 to 23,
the vector is a viral vector, and the viral vector is selected from among a retrovirus, a lentivirus, an adenovirus, an adeno-associated virus (AAV), a vaccinia virus, a poxvirus, a human immunodeficiency virus (HIV), a murine leukemia virus (MLV), an Avian sarcoma/leukosis (ASLV), a SNV (Spleen necrosis virus), a Rous sarcoma virus (RSV), a Mouse mammary tumor virus (MMTV), a Herpes simplex virus, an episomal, and a herpes simplex virus.

### Embodiment 28: Conversion method (1)

A method for direct cell-conversion of a somatic sell into a motor neuron,
in which the direct cell-conversion is that the somatic cell is directly converted into an induced motor neuron (iMN),
in which the method includes:
   a) introducing Embodiment 1, Embodiment 16, or Embodiment 20 into the somatic cell.

### Embodiment 29: Conversion method (1)

In Embodiment 28,
the method further includes
b) introducing one embodiment selected from Embodiments 2 to 9 and 21 to the somatic cells.

### Embodiment 30: Conversion method (1)

In Embodiment 28 or Embodiment 29,
after the introduction,
the method further includes: culturing the somatic cell using a medium for neural differentiation.

### Embodiment 31: Conversion method (2)

A method for direct cell-conversion of a somatic sell into a motor neuron,
in which the direct cell-conversion is that the somatic cell is directly converted into an induced motor neuron (iMN),
in which the method includes:
   (a) mixing Embodiment 1, Embodiment 16, or Embodiment 20 with a culture medium; and
   (b) culturing somatic cells using the mixture.

### Embodiment 32: Conversion method (3)

A method for direct cell-conversion of a somatic sell into a motor neuron,
in which the direct cell-conversion is that the somatic cell is directly converted into an induced motor neuron (iMN),
in which the method includes:
   introducing Embodiment 11, Embodiment 19, or Embodiment 22 into the somatic cell;
      or
   introducing Embodiment 16, Embodiment 17, or Embodiment 18 into the somatic cell.

### Embodiment 33: Introduction means

In any one embodiment of Embodiments 28 to 32,
the instruction is performed by a method selected from among electroporation, gene gun, sonoporation, magnetofection, microinjection, transient cell compression or squeezing, cationic liposomal method, lithium acetate-DMSO, lipid-mediated transfection, phosphoric acid Calcium precipitation, lipofection, polyethyleneimine (PEI)-mediated transfection, and DEAE-dextran-mediated transfection.

### Embodiment 34: Type of somatic cell

In any one embodiment of Embodiments 28 to 32,
the somatic cell is any one selected from among a fibroblast, an epithelial cell, an endothelial cell, a muscle cell, a nerve cell, a cell derived from hair, a cell derived from hair root, a hair follicle cell, an oral epithelial cell, a somatic cell extracted from urine, a gastric mucosal cell, a goblet cell , a gastrin cell/G cell, a B cell, a pericyte cell, an astrocyte, a blood cell, and an oligodendrocyte progenitor cell.

### Embodiment 35: Conversion period

In any one embodiment of Embodiments 28 to 32,
the method is characterized in that the somatic cells are converted into motor neurons within 1 to 10 weeks by the introduction.

### Embodiment 36: Characteristics of conversion method

In any one embodiment of Embodiments 28 to 32,
the method is characterized in that by the introduction, the somatic cells become induced motor neurons without generating induced stem cells (iPSCs).

### Embodiment 37: Product of conversion method - motor neuron (over-expression of converting factor)

A motor neuron in which a direct cell-conversion factor is over-expressed,
in which the direct cell-conversion factor is one or more selected from among Gsta4, Ascl1 (Achaete-scute homolog 1), Brn2 (POU Class 3 Homeobox 2), Myt1L (Myelin Transcription Factor 1 Like), Hb9 (Homeobox HB9), ISL1 (ISL LIM homeobox 1), Lhx3 (LIM homeobox 3), Ngn2 (neurogenin 2), and NeuroD1 (neuronal differentiation 1), and
the motor neuron is a motor neuron produced by direct cell-conversion of a somatic cell.

### Embodiment 38: Product of conversion method - motor neuron (over-expression of motor neuron)

A motor neuron in which neuronal markers are over-expressed,
in which the neuronal markers are one or more selected from among NEUN, ChAT, Map2, Hb9, Synapsin, Tuj1, IsL1, PAX6, Olig2, Nkx2.2, and SMI-32, and
the motor neuron is a motor neuron produced by direct cell-conversion of a somatic cell.

### Embodiment 39: Pharmaceutical composition (1)

A pharmaceutical composition for preventing or treating a nervous system disease, the composition including
Embodiment 1 or 16 or 20.

### Embodiment 40: Pharmaceutical composition (2)

A pharmaceutical composition for preventing or treating a nerve system disease, the composition including:
Embodiment 1 or 16 or 20; and
one embodiment selected from among Embodiments 2 to 9 and Embodiment 21.

### Embodiment 41: Pharmaceutical composition (3)

A pharmaceutical composition for preventing or treating a nerve system disease, the composition including
Embodiment 37 or 38.

### Embodiment 42: Indications

In any one embodiment of Embodiments 39 to 41,
the nervous system disease is one or more selected from Spinal cord injury, Parkinson's disease, stroke, Huntington's disease, Lou Gehrig's disease, Ataxia telangiectasia, amyotrophic lateral sclerosis, motor neuron damage, traumatic peripheral nerve damage, ischemic brain injury, neonatal hypoxic ischemia Brain damage, cerebral palsy, peripheral palsy, central paralysis, quadriplegia, biparesis, epilepsy, neuronal development disorder, neuralgia, intractable epilepsy, Alzheimer's disease, congenital metabolic nervous system disease, traumatic brain injury, motor nerve cell damage, and a disease caused by the motor nerve cell damage.

### Embodiment 42: Extra component

In any one embodiment of Embodiments 39 to 41,
the pharmaceutical composition is characterized in that it further includes at least one selected from an acceptable carrier, excipient, diluent, and preservative.

### Embodiment 43: Treatment method (1)

A method for treating a nervous system disease, the method including:
administering Embodiment 39 or 40 to a subject,
in which the somatic cells become induced motor neurons without generating induced stem cells (iPSCs).

### Embodiment 44: Treatment method (2)

A method for treating a nervous system disease, the method including:
administering somatic cells introduced with Embodiment 39 or 40 to a subject,
in which the somatic cells become induced motor neurons without generating induced stem cells (iPSCs).

### Embodiment 45: Treatment method (3)

A method for treating a nervous system disease, the method including:
administering Embodiment 41 to a subject,
in which the somatic cells in the subject become induced motor neurons without generating induced stem cells (iPSCs).

### Embodiment 46: Subject restriction (1)

In any one embodiment of Embodiments 43, 44, and. 45,
the subject is a mammal.

### Embodiment 47: Subject restriction (2)

In Embodiment 46,
the mammal is any one selected from humans, mice, dogs, and cats.

### Embodiment 48: Administration restriction

In any one embodiment of Embodiments 43, 44, and 45,
the administration site is selected from muscle, intradermal, subcutaneous, intravenous, abdominal, arterial, mucosal, spinal, bone marrow, intrathecal, and transdermal.

### Embodiment 49: Dosage

In any one embodiment of Embodiments 43, 44, and 45,
the administration is performed at a dose of 1 uL/kg to 20 uL/kg at a time.

### Embodiment 50: Indications

In any one embodiment of Embodiments 43, 44, and 45,
the nervous system disease is one or more selected from Spinal cord injury, Parkinson's disease, stroke, Huntington's disease, Lou Gehrig's disease, Ataxia telangiectasia, amyotrophic lateral sclerosis, motor neuron damage, traumatic peripheral nerve damage, ischemic brain injury, neonatal hypoxic ischemia Brain damage, cerebral palsy, peripheral palsy, central paralysis, quadriplegia, biparesis, epilepsy, neuronal development disorder, neuralgia, intractable epilepsy, Alzheimer's disease, congenital metabolic nervous system disease, traumatic brain injury, motor nerve cell damage, or a disease caused by the motor nerve cell damage.

### Mode of the Invention

### Mode of the Invention

Hereinafter, the present invention will be described in more detail with reference to examples.

These examples are presented only for illustrative purposes, and it will be apparent to those ordinarily skilled in the art that the scope of the present invention is not to be construed as being limited by these examples.

### Experimental material

### • Vector designing

An AAV vector (Cell Biolabs, INC., VPK-402) was used as a vector in examples described below, and a schematic diagram of the AAV vector for introducing converting factors is shown in FIG. 1.

FIG. 1(a) corresponds to SEQ ID NO: 36, FIG. 1(b) corresponds to SEQ ID NO: 3, FIG. 1(c) corresponds to SEQ ID NO: 4, FIG. 1(d) corresponds to SEQ ID NO: 5, and FIG. 1(e) corresponds to SEQ ID NO: 29.

### • AAV production

The 293T cell line was used to construct AAV. 293T cells were cultured in Dulbecco's Modified Eagle Medium (DMEM, Thermal Fisher, #12430112) added with 10% fetal bovine serum (Thermal Fisher, #26140079) and 1% antibiotic-antimyotic (Thermal Fisher, #15240096) under conditions of 5% CO₂ and 37°C. The 293T cells were transfected with the AAV vector of FIG. 1, pHelper, and pRapCap, and cultured for 48 hours. Thereafter, viruses containing converting factors were extracted through a virus extraction process.

### • Cell culturing and AAV introduction into cells

Human dermal fibroblasts (Sigma, 106-05A) were cultured in fibroblast growth medium (Sigma, 116-500) added with 10% fetal bovine serum (Thermal Fisher, #26140079) and 1% antibiotic-antimyotic (Thermal Fisher, #15240096) under conditions of 37°C and 5% CO₂.

The human dermal fibroblasts were seeded at a concentration of 0.025 x 10⁶ cells per well of 24 wells. In 24 hours after the cell seeding, the cells were treated with converting factors. After 24 hours of converting factor treatment, the culture medium was replaced with a fibroblast growth medium. After another 24 hours, the medium was replaced with a neural induction medium. The medium was replaced every other day. On the 14th to 17th day, the cells were subjected to immunofluorescence staining through fixation.

Mouse astrocytes (Abm, T0289) were cultured in Prigrow III medium (Abm, TM003) added with 10% fetal bovine serum (Thermal Fisher, #26140079) and 1% Penicillin/Streptomycin (Gibco) under conditions of 37°C and 5% CO₂.

The mouse astrocytes were seeded at a concentration of 0.025 x 10⁶ cells per well of 24 wells. In 24 hours after the cell seeding, the cells were treated with converting factors. After another 24 hours, the medium was replaced with Prigrow III medium. After another 24 hours, the medium was replaced with Neural induction medium. The medium was replaced every other day. On the 14th to 17th day, the cells were subjected to immunofluorescence staining through fixation.

Mouse embryonic fibroblasts were cultured in DMEM (Gibco) added with 10% fetal bovine serum (Thermal Fisher, #26140079) and 1% Penicillin/Streptomycin (Gibco) under conditions of 37°C and 5% CO₂.

The mouse embryonic fibroblasts were seeded at a concentration of 0.025 x 10⁶ cells per well of 24 wells. In 24 hours after the cell seeding, the cells were treated with converting factors. After another 24 hours, the medium was replaced with DMEM medium. After another 24 hours, the medium was replaced with Neural induction medium. The medium was replaced every other day. On the 14th to 17th day, the cells were subjected to immunofluorescence staining through fixation.

When AAV was introduced into the SCI model, the AAV was microinjected into the damaged area (Lumbar 5) of the mouse spinal cord injury model using an azimuth fixation device (Stereotaxic, Harvard Apparatus Co.), and analysis was performed for 60 days.

### • Immunofluorescence staining

For cell fixation, the cells were washed twice for 5 minutes each with PBS (Gibco). Next, 4% paraformaldehyde (ThermoFisher) was added, followed by incubation at room temperature for 10 minutes.

For permeabilization, PBS 0.10 + Triton-X was added, followed by incubation at room temperature for 10 minutes. The cells were washed 3 times, each time, with PBS for 5 minutes.

For blocking and immunostaining, the mixture of 1% BSA + PBST (PBS + 0.1% Tween 20) was added, followed by incubation at room temperature for 30 minutes. As primary antibodies, ChAT (Invitrogen, 1:1000), Map2 (Millipore, 1:200), and NeuN (Merck Millipore, 1:100) were used. The primary antibodies were added to the mixture of 1% BSA + PBST and was left at 4°C overnight. After washing with PBS for 5 minutes, three times, a secondary antibody, Alexa FluorTM 488/594 (ThermoFisher, 1:1000), was added to the mixture of 1% BSA + PBST, followed by incubation at room temperature for 1 hour in a dark place. After washing with PBS for 5 minutes three times, DAPI staining was performed.

### • RNA extraction and cDNA synthesis

2 x 10⁶ cells were isolated with trypsin (ThemoFisher), and then centrifuged at 13,000 rpm for 10 seconds. After the removal of the supernatant, 1 ml of easy-BlueTM (iNtRON) was added, the mixture was vortexed for 10 seconds, and then 200 µl of choloroform was added and vortexed. After centrifugation at 13,000 rpm for 10 minutes, 400 µl of an upper fluid was transferred to a new 1.5-ml tube. After adding 400 µl of 2-propanol (Sigma), followed by 2 to 3 times of inverting, followed by placing at room temperature for 10 minutes. After centrifugation at 13,000 rpm for 5 minutes, the upper layer was removed. 1 ml of 75% EtOH (Sigma) was added, followed by 2 to 3 times of inverting. Centrifugation was performed at 10,000 rpm at 4°C 5 minutes, and the upper layer was removed. After drying at RT for 5 minutes, RNA was dissolved by adding 20 µl of distilled water. The end products obtained were stored at -70°C.

cDNA was synthesized using AccuPower^{®}CycleScript^{™} RT PreMix & Master Mix (Bioneer).

### • Real-time quantitative reverse transcription PCR (qRT-PCR)

It was performed using qPCR (SYBRGreen Realtime PCR Master Mix, TOYOBO). In human fibroblasts converted into motor neurons induced qRT-PCR, expression of Synapsin, Map2, and Hb9 was confirmed. In mouse-derived fibroblasts converted into motor neurons, expression of Synapsin, Map2, and Hb9 was confirmed.

The primers used here are shown in the table below.

**[Table 1]**

| **Name** | **Sequence** | **SEQ ID NO:** |
|---|---|---|
| hSYN1_F | GTG GCA CTG ACC AAG ACG TA | SEQ ID NO: 6 |
| hSYN1_R | CTG ACG TCC TCA TGT AGG CC | SEQ ID NO: 7 |
| hMAP2_F | TCC TCC ATT CTC CCT CCT CG | SEQ ID NO: 8 |
| hMAP2_R | CGA ATT GGC TCT GAC CTG GT | SEQ ID NO: 9 |
| hHb9_F | GAG CTT GAA CTG GTG CTC CA | SEQ ID NO: 10 |
| hHb9_R | TCT CAA CAG TAG GTG CCT GC | SEQ ID NO: 11 |
| mMap2-F | ACC TTC CTC CAT CCT CCC TC | SEQ ID NO: 30 |
| mMap2-R | TCC TGC TCT GCG AAT TGG TT | SEQ ID NO: 31 |
| mHb9-F | TCC AGA ACC GCC GAA TGA AA | SEQ ID NO: 32 |
| mHb9-R | CCA TCA GCT CCT CTT CCG TC | SEQ ID NO: 33 |
| mSynapsin-F | TCC TCC TGC TCA ACA ACG AC | SEQ ID NO: 34 |
| mSynapsin-R | CGG TCC AAG GCC AGA AAG AT | SEQ ID NO: 35 |

### Example 1: Examination of direct cell-conversion of Gsta4 into motor neurons in cells

Gsta4 was introduced into mouse-derived fibroblasts using AAV. Conversion of somatic cells into nerve cells by Gsta4 was examined by immunostaining fluorescence and qPCR (see FIG. 2). Expression of ChAT, which is a motor neuron marker, and Map2, which is a mature neuron marker, were examined by immunofluorescence staining. With the use of qPCR, the degree of conversion of neurons was examined by changes in the expression of Synapsin, which is a marker for synapse formation, Map2, which is a marker for mature neurons, and Hb9, which is a marker for mature neurons.

When treated with Gsta4 solely, the expression level of synapsin was determined to be increased about 4 times compared to the control group (marked as control), Map2 was determined to be increased about 5 times compared to the control group (marked as control), and Hb9 was determined to be increased about 2 times compared to the control group (marked as control). On the basis of these experimental results, it was confirmed that effective conversion into neurons from somatic cells can be achieved with the use of Gsta4 solely.

### Example 2: Verification of direct cell-conversion of known converting cells into motor neurons in cells

To examine the direct cell-conversion effect of known factors, the degree of direct cell-conversion into motor neurons by introduction of known converting factors into human-derived fibroblasts was examined (FIG. 3).

The known converting factors were introduced in a manner described below.

Administration of Ascl1 alone; administration of an Ascl1, Brn2, and Myt11 combination (marked as ABM); administration of an Hb9, Isl1, and Lhx3 combination (marked as HIL); administration of an ABM and HIL combination; administration of an ABM, HIL, Ngn2, and NeuroD1 combination (marked as HND).

It was confirmed that when treated with Gsta4 alone as in Example 1, the expression of Synapsin, Map2, and Hb9 was increased by 4 times, 5 times, and 2 times compared to the control group. In contrast, when treated with Ascl1 alone, the expression of Synapsin, Map2, and Hb9 was only about 1 to 1.5 times. This suggests that compared to the administration of a known factor, Ascl1, alone, when treated with Gsta4, which is a novel converting factor of the present application, alone, efficiency of direct cell-conversion into motor neurons is high.

In addition, upon administration of ABM in which three known factors were combined, the expression of Synapsin, Map2, and Hb9 increased by only about 2 times, 4 times, and 1 time. In addition, upon administration of HIL in which three known factors were combined, the expression of Synapsin, Map2, and Hb9 increased only about 2 times, 1.5 times, and 1.5 times. Through this, it was confirmed that even when the three known factors were administered in combination, the efficiency of direct cell-conversion into motor neurons was lower than or similar to that of the case of the treatment with Gsta4 alone, which is a novel differentiation factor of the present application.

Administration of ABM+HIL in which six known factors are combined; when ABM+HND in which six known factors were combined was administered, it was confirmed that the expression of neuronal markers increased compared to the case where one known factor was administered, or the case where 3 known factors were together. That is, the case of combining 6 or more factors exhibited a significant effect.

### Example 3: Examination of direct cell-conversion according to combined use of Gsta4

After confirming the neuronal conversion effect of only the known factors in Example 2, the effect of the combined use of Gsta4 of the present invention and known factors were examined.

To this end, AAV was introduced into mouse-derived fibroblasts by diversely combining Gsta4 and conventional converting factors, and the degree of conversion was examined by the expression of neuronal markers (Synapsin, Map2, and Hb9) through qPCR (FIG. 4).

As a result, it was confirmed in the group in which Gsta4 and the conventional converting factors were combined, the expression of neuronal markers increased about 5 to 10 times compared to the control group (marked as Control). Compared to the case where only the conventional factors were used as in Example 2, it was found that the fold change of each marker was higher when Gsta4 of the present invention was combined.

Expression of ChAT, which is a motor neuron marker, and Map2, which is a mature neuron marker, were examined by immunofluorescence staining (FIGS. 5 and 6). Through the immunofluorescence staining, it was confirmed that the expression of ChAT and Map2 appeared in the groups in which Gsta4 and conventional converting factors were used together.

That is, it was found from FIGS. 4 to 6 that somatic cells were effectively directly converted into motor neurons even when Gsta4 and conventional converting factors were used together.

### Example 4: Examination of direct cell-conversion of conventional converting factors into motor neurons in cells according to presence/absence of Gsta4

After confirming the results of Examples 2 and 3, the effect of combining Gsta4 of the present invention with known converting factors was examined in more detail.

### Comparison between use of one conventional converting factor and combined use of one conventional converting factor and Gsta4

With the use of AAV, conventional converting factors with or without Gsta4 were introduced into mouse-derived fibroblasts, and the degree of conversion was examined by the expression of neuronal markers (Synapsin and Map2) through qPCR (FIG. 7).

Compared to the case where each of Ascl1 and Brn2 was individually administered, it was confirmed that when Gsta4 and Ascll (marked as Gsta+Ascl1) were administered together and when Gsta4 and Brn2 (marked as Gsta4+Brn2) were administered together, the expression of neuronal markers (Synapsin and Map2) increased about 2 to 5 times.

### Comparison between use of two conventional converting factors and combined use of two conventional converting factors and Gsta4

With the use of AAV, conventional converting factors with or without Gsta4 were introduced into mouse-derived fibroblasts, and the degree of conversion was examined by the expression of neuronal markers (ChaT and Map2) through immunofluorescence staining (see FIG. 8).

Compared to the case where Mnx1 and Lhx3 were used together (marked as Mnx1+Lhx3), it was confirmed that when Gsta4, Mnx1, and Lhx3 were used together (marked as Gsta4+Mnx1+Lhx3), the number of neuronal markers stained by immunostaining increased by about 40 times.

In addition, the expression of neuronal markers (Synapsin, Map2) was examined by qPCR (FIG. 9).

Compared to the case where Mnx1 and Lhx3 which were conventional converting factors were used together (marked as Mnx1+Lhx3), it was confirmed that when Gsta4, Mnx1, and Lhx3 were used together (marked as Gsta4+Mnx1+Lhx3), the expression of neuronal markers increased about 2 to 4 times. In addition, when Gsta4 was administered alone (marked as Gsta4), the expression of neuronal markers increased about 2 to 4 times compared to the control group (marked as Control).

### Comparison between use of three conventional converting factors and combined use of three conventional converting factors and Gsta4

With the use of AAV, conventional converting factors with or without Gsta4 were introduced into mouse-derived fibroblasts, and the degree of conversion was examined by the expression of neuronal markers (Synapsin and Map2) through qPCR (FIG. 10).

Compared to the case where Ascl1, Brn2, and Mytll which were conventional converting factors were administered together (marked as Ascl1+Brn2+Myt1l), it was confirmed that when Gsta4, Ascl1, Brn2, and Mytll were administered together (marked as Gsta4+Ascl1+Brn2+Myt1l), the expression of neuronal markers increased about 2 to 4 times.

### Comparison between use of eight conventional converting factors and combined use of eight conventional converting factors and Gsta4

With the use of AAV, conventional converting factors with or without Gsta4 were introduced into human-derived fibroblasts, and the degree of conversion was examined by the expression of neuronal markers (Synapsin, Map2, and Hb9) through qPCR (FIG. 11).

Compared to the case where Ascl1, Brn2, Myt1l, Hb9, Isl1, Lhx3, Ngn2, and NeuroD1, which were conventional converting factors, were administered in combination (marked as ABM+HIL+Ngn2+NeuroD1(HND)), it was confirmed that when Gsta4 was administered in combination with Ascl1, Brn2, Myt1l, Hb9, Isl1, Lhx3 , Ngn2, and NeuroD1 (marked as ABM+HND+Gsta4), the expression of neuronal markers increased about 2 to 5 times. In addition, when Gsta4 was administered in combination with Ascl1, Brn2, Myt1l, Hb9, Isl1, Lhx3, Ngn2, and NeuroD1, the expression of neuronal markers increased about 10 to 12 times compared to the control group (marked as Control).

With the use of AAV, conventional converting factors with or without Gsta4 were introduced into human-derived fibroblasts, and the degree of conversion was examined by the expression of neuronal markers (vChaT and Map2) through immunofluorescence staining (see FIG. 13).

When the conventional converting factors, Ascl1, Brn2, Mytll, Hb9, Isl1, Lhx3, Ngn2, and NeuroD1, were introduced together (marked as known factors), about 15.51% of the cells were differentiated into mature neurons. In contrast, it was confirmed that about 48.72% of the cells were differentiated into mature neurons when the known factors and Gsta4 were introduced together (marked as known factors+Gsta4).

### Comparison of use of four conventional converting factors vs. use of seven conventional converting factors vs. combined use of seven conventional converting factors and Gsta4

With the use of AAV, conventional converting factors with or without Gsta4 were introduced into mouse-derived fibroblasts, and the degree of conversion was examined by the expression of neuronal markers (vChaT and Map2) through immunofluorescence staining (see FIG. 12).

When Hb9, Isl1, Lhx3, and Ascl1, which were conventional converting factors, were administered together, about 20% of neuronal markers were expressed. When Ascl1, Brn2, Myt1l, Hb9, Isl1, Lhx3, and Ngn2, which were conventional converting factors, were administered together (marked as known factors), about 50% of neuronal markers were expressed. On the other hand, when the known factors and Gsta4 were administered in combination (marked as known factors+Gsta4), about 70% of neuronal markers were expressed. That is, it was found that the expression of ChaT and Map2 increased when Gsta4 and 7 conventional converting factors were administered in combination compared to the case where only 7 conventional converting factors were administered.

From the above results, it was found that Gsta4 of the present application is a converting factor having a function of converting somatic cells such as fibroblasts and astrocytes into induced motor neurons through direct cell-conversion. In addition, it was found that Gsta4 has higher conversion efficiency than conventional direct cell-conversion factors. In addition, it was found that when the conventional converting factors and Gsta4 were used together, the conversion efficiency of conventional converting factors was increased.

### Example 5: Examination of direct cell-conversion into motor neurons in vivo and treatment effect

On the basis of the results of Examples 1 to 4 in which the neuronal conversion efficacy of Gsta4 in cells was examined, an experiment according to the schematic diagram of FIG. 14 was performed to examine the direct cell-conversion into motor neurons in vivo and the therapeutic effect.

In the SCI mouse model, which is an animal model for paraplegic disease, the effect of improving or treating spinal nerve damage was examined through morphological, physiological, and behavioral analyses.

### 1) Creation of Spinal cord injury (SCI) mouse model

To create an SCI mouse model, which is a paraplegic model due to spinal cord injury, ICR mice were anesthetized, and the Lumbar 5 region of the spinal cord (hereinafter referred to as L5, corresponding to spinal column T13-L1) of each mouse was damaged. After 5 weeks of damage, the mice were used for the experiment.

### 2) AAV administration to SCI mouse model

2 ul of AAV was micro-injected into the L5 region of the SCI mouse model using a micro-syringe (Hamilton, 705).

### Example 5-1: Examination of morphological change in spinal cord injury site

To examine the morphological change in the spinal cord injury site (L5) for each of a group (referred to as Control) in which 8 conventional converting factors (Ascl1, Brn2, Myt1l, Hb9, Isl1, Lhx3, Ngn2, and NeuroD1) were introduced into the SCI mouse model, a group (referred to as +Gsta4) in which 8 conventional converting factors and Gsta4 were introduced together, and a group (referred to as as Mock) in which AAV with no converting factors was administered, crystal violet (Sigma) staining was performed (see FIG. 15).

Referring to FIG. 15, in the Mock group in which no direct cell-conversion factors were administered, many inflammatory cells gathered to form thick scars (red dotted line) . In the Control group in which 8 conventional converting factors were administered, the number of scars was reduced compared to the Mock group. On the other hand, in the group in which 8 conventional converting factors and Gsta4 were administered together, it was confirmed that scars caused by inflammatory cells were alleviated compared to the Control group.

### Example 5-2: Examination of changes in expression of motor neuron markers

To examine the changes in the expression of motor neuron markers in the spinal cord injury site (L5) for each of a group (referred to as Control) in which 8 conventional converting factors (Ascl1, Brn2, Myt1l, Hb9, Isl1, Lhx3, Ngn2, and NeuroD1) were introduced into the SCI mouse model, a group (referred to as +Gsta4) in which 8 conventional converting factors and Gsta4 were introduced together, and a group (referred to as Mock) in which AAV with no converting factors was administered, immunofluorescence staining was performed (see FIGS. 16, 17, and 18) .

Referring to FIGS. 16, 17, and 18, the expression of ChaT and Map2 in the Control group increased about 2 times compared to the Mock group. On the other hand, in the group in which 8 conventional converting factors and GSTA4 were introduced, the expression of ChaT and Map2 increased about 5 times or more compared to the Control group. This means that the efficiency of direct cell-conversion of astrocytes into motor neurons was higher than the Control group.

### Example 5-3: Examination of treatment effect of paraplegia through electrophysiological analysis

Electrophysiological changes were examined in the spinal cord injury site (L5) (see FIG. 19) for a normal group (referred to as Sham) without damage to the spinal cord, a group (referred to as Control) in which AAV introduced with 8 conventional converting factors (Ascl1, Brn2, Myt1l, Hb9, Isl1, Lhx3, Ngn2, NeuroD1) was administered to the SCI mouse model; a group (referred to as +Gsta4) in which AAV introduced with 8 conventional converting factors and Gsta4 was administered, and a group (referred to as Mock) in which AAV was introduced into the SCI mouse model.

FIG. 19(a) is a photograph of a neuron having undergone patch clamping.

Referring to the action potential measurement results of FIG. 19(b), the action potential in the Sham group was 25 Hz, but the action potential in the Mock group was reduced, by more than 1/10, to 2 Hz. On the other hand, in the control group introduced with 8 conventional converting factors, the action potential increased to 6 Hz, and in the group introduced with 8 conventional converting factors and Gsta4, the action potential increased to 13 Hz, which was two times the action potential of the Control group.

An increase in action potential means the ability of a nerve cell to transmit a signal when it receives a signal. Signal transduction cannot occur without an action potential. The action potential is also the most important characteristic of neurons. The fact that the action potential is generated and increased means that the conversion into neurons has been done well. Therefore, on the basis of the fact that the group introduced with 8 converting factors and Gsta4 showed the best efficiency of conversion into neurons, the combination has good conversion efficiency for conversion into neurons.

Referring to the results of measuring the signal from the pre-synaptic nerve cell in FIG. 19(c), the Mock group exhibited 1 Hz, the Control group exhibited 6 Hz, and the +Gsta4 group exhibited 47 Hz.

An increase in signals coming from pre-synaptic neurons indicates an increase in signal transmission between neurons. This indicates that the neuronal disconnections caused by SCI is removed, and the neurons were reconnected by generation of new motor neurons.

### Example 5-4: Examination of direct cell-conversion through behavioral analysis

The change in BBB score was examined in the spinal cord injury site (L5) (see FIG. 20) for a group (referred to as Sham) with no spinal cord injury, a group (referred to as -Gsta4) in which 8 conventional converting factors (Ascl1, Brn2, Myt1l, Hb9, Isl1, Lhx3, Ngn2, NeuroD1) were introduced into the SCI mouse model, a group (referred to as +Gsta4) in which eight conventional converting factors and Gsta4 were introduced, and a group (referred to as Mock) in which AAV was administered to the SCI mouse model.

The BBB score (Basso Beattie, and Bresnahan score) in FIG. 20 is a measurement method traditionally used in spinal cord injury models, and the scores are obtained by comprehensively determining the shape and angle of the lower extremity, the angle of the knee, the shape, angle, and position of the sole, etc. The lowest score is 0 and the maximum score is 21 points, with normal animals scoring 21 points.

The group (-Gsta4) introduced with only 8 conventional converting factors showed no difference from the Mock group (BBB score of about 4 points). However, the group (+Gsta4) introduced with 8 conventional converting factors and Gsta4 showed an increase of about 2-fold or more compared to the -Gsta4 group on the third week (BBB score of about 8 points) after the AAV administration.

In addition, changes in each of the forced swimming test, self-urination, and foot printing test were exampled (see FIG. 21) for a normal group (Sham) without damage to the spinal cord, a group (Mock) in which AAV was introduced into the SCI mouse model, a group (+Gsta4) in which AAW introduced with 8 conventional converting factors and Gsta4 was administered, and a group (Control) in which AAV introduced with 8 conventional converting factors was administered.

In the forced swimming test of FIG. 21(a), the animal model was placed in a water bath for 3 minutes and the number of movements of the lower extremities was counted. In the Mock group, the lower extremities were immobilized due to spinal cord injury. However, when 8 conventional converting factors and Gsta4 were introduced together, it was confirmed that the movement of the lower extremities increased by about 6 to 12 times.

FIG. 21(b) and FIG. 21(c) are the results of measuring self-urination ability. Mice with spinal cord injury cannot urinate on their own. It was confirmed that in the case of the group in which AAV introduced with conventional converting factors and Gsta4 was administered, the mice urinated on their own on Day 14 or 16 after the administration.

FIG. 21(d) is a result of a foot printing test. Foot printing is an experiment in which red ink is smeared on the soles of the lower legs of a mouse and the shape of the soles is examined. In the case of the Sham group, the soles of the lower extremities were clearly stamped (11 footprints/20 cm), but in the Mock group, the shape of the soles could not be identified (11 footprints/20 cm). On the other hand, in the group introduced with 8 conventional converting factors and Gsta4, it was confirmed that the soles of the mice were imprinted (5 footprints/20 cm).

From the above results, it was found that Gsta4, the novel differentiation factor discovered by the inventors of the present application, was an important factor in the direct cell conversion of somatic cells into motor neurons. In addition, Gsta4 has higher conversion efficiency than conventional converting factors. Furthermore, when conventional converting factors and Gsta4 are used together, conversion efficiency is increased compared to the case only conventional converting factors are used. Furthermore, the inventors also confirmed the therapeutic effect of a paraplegic model with spinal cord injury, and Gsta4 is expected to play an important role in various nervous system diseases in the future.

### Industrial Applicability

The present application provides a novel converting factor for direct cell-conversion of a somatic cell to a motor neuron and uses thereof.

### Sequence List Text

This relates to glutathione S-transferase alpha 4 (Gsta4) protein or a nucleic acid sequence encoding the same.

## Claims

1. A composition for a direct cell-conversion, comprising:
a Gsta4(Glutathione S-Transferase Alpha 4) protein or a nucleic acid encoding the same,
wherein the direct cell-conversion is that a somatic cell is directly converted into an iMN (induced motor neuron).

2. The composition of claim 1,
wherein the Gsta4 protein is an amino acid sequence represented by SEQ ID NO: 1 or an amino acid sequence having a of 70% or more sequence identity thereto.

3. The composition of claim 1,
wherein the nucleic acid encoding Gsta4 protein has a sequence of SEQ ID NO: 1 or 12.

4. The composition of claim 1,
wherein the composition further comprises
ii) at least one protein selected from Ascl1 (Achaete-scute homolog 1), Brn2 (POU Class 3 Homeobox 2), MytlL (Myelin Transcription Factor 1 Like), Hb9 (Homeobox HB9), ISL1 (ISL LIM homeobox 1), Lhx3 (LIM homeobox 3), Ngn2 (neurogenin 2) and NeuroD1 (neuronal differentiation 1);
or a nucleic acid encoding the selected at least one protein.

5. The composition of claim 4,
wherein the selected protein is Hb9,Lhx3 orNgn2.

6. The composition of claim 4,
wherein the selected proteins are
Hb9 and Lhx3; Hb9 and Ngn2; or Lhx3 and Ngn2.

7. The composition of claim 4,
wherein the selected proteins are Hb9, Lhx3 and Ngn2.

8. The composition of claim 4,
wherein the selected proteins are Hb9, Lhx3, Ngn2 and Isl1.

9. The composition of claim 4,
wherein the selected proteins are Ascl1, Brn2, Myt1L, Hb9, Isl1, Lhx3, Ngn2 and NeuroD1.

10. The composition of claim 4,
Ascl1 protein is SEQ ID NO: 17, a nucleic acid encoding Ascl1 protein comprises SEQ ID NO: 18,
Brn2 protein is SEQ ID NO: 19, a nucleic acid encoding Brn2 protein comprises SEQ ID NO: 20,
MytlL protein is SEQ ID NO: 21, a nucleic acid encoding MytlL protein comprises SEQ ID NO: 22,
Hb9 protein is SEQ ID NO: 13, a nucleic acid encoding Hb9 protein comprises SEQ ID NO: 14,
Isl1 protein is SEQ ID NO: 23, a nucleic acid encoding Isl1 protein comprises SEQ ID NO: 24,
Lhx3 protein is SEQ ID NO: 15, a nucleic acid encoding Lhx3 protein comprises SEQ ID NO: 16,
Ngn2 protein is SEQ ID NO: 25, a nucleic acid encoding Ngn2 protein comprises SEQ ID NO: 26,
NeuroD1 protein is SEQ ID NO: 27, a nucleic acid encoding NeuroD1 protein comprises SEQ ID NO: 28.

11. A vector for expressing a converting factor for a direct cell-conversion, comprising:
i) a nucleic acid encoding Gsta4(Glutathione S-Transferase Alpha 4) protein; and
ii) a promoter,
wherein the ii) is operably linked to the i),
wherein the direct cell-conversion is that a somatic cell is directly converted into an iMN (induced motor neuron).

12. The vector of claim 11,
wherein the vector further comprises
iii) a nucleic acid encoding at least one protein selected from Ascl1, Brn2, Myt1L, Hb9, Isl1, Lhx3, Ngn2 and NeuroD1.

13. The vector of claim 12,
wherein the selected proteins are Hb9 and Lhx3.

14. A vector for expressing a converting factor for a direct cell-conversion,
the vector comprises a first vector and a second vector,
the first vector comprising:
i) a nucleic acid encoding Gsta4(Glutathione S-Transferase Alpha 4) protein; and ii) a promoter which is operably linked to the i),
the second vector comprising:
iii) a nucleic acid encoding at least one protein selected from Ascl1, Brn2, Myt1L, Hb9, Isl1, Lhx3, Ngn2 and NeuroD1; and iv) a promoter which is operably linked to the iii),
wherein the ii) and the iv) are same or different,
wherein the iii) included in one vector or in two or more vectors,
wherein the direct cell-conversion is that a somatic cell is directly converted into an iMN (induced motor neuron).

15. A vector of any one of claims 11 to 14,
wherein the vector further comprises one or more selected from
an enhancer, a polyadenylation signal, a Kozak consensus sequence, an inverted terminal repeat(ITR), a long terminal repeat(LTR), a terminator, an internal ribosome entry site(IRES), fluorescent protein gene, a glutathione-S-transferase(GST), a horseradish peroxidase(HRP), a chloramphenicol acetyltransferase (CAT) beta-galactosidase, a beta-glucuronidase, a luciferase, a histidine (His) tag, a V5 tag, a FLAG tag, an influenza hemagglutinin (HA) tag, a Myc tag, a 2A self-cleaving peptide, and an antibiotic resistance gene.

16. The vector of claim 15,
wherein the 2A self-cleaving peptide is selected from T2A, P2A, E2A and F2A.

17. The vector of any one of claims 11 to 14,
wherein the vector is a viral vector or a non-viral vector,
wherein the viral vector is selected from a retrovirus, a lentivirus, an adenovirus, an adeno-associated virus (AAV), a vaccinia virus, a poxvirus, a human immunodeficiency virus (HIV), a murine leukemia virus (MLV), an Avian sarcoma/leukosis (ASLV), a SNV (Spleen necrosis virus), a Rous sarcoma virus (RSV), a Mouse mammary tumor virus (MMTV), a Herpes simplex virus, an episomal, and a herpes simplex virus.

18. A method for directly converting a somatic cell into an iMN (induced motor neuron), comprising:
introducing a composition comprising a Gsta4 protein or a nucleic acid encoding the Gsta4 protein into a somatic cell;
wherein the introduction induces that a somatic cell is directly converted into an iMN (induced motor neuron) without generating iPSCs (induced pluripotent stem cells).

19. The method of claim 18,
wherein the somatic cell is one or more selected from a fibroblast, an epithelial cell, an endothelial cell, a muscle cell, a nerve cell, a hair cell, a hair root cell, a hair follicle cell, an oral epithelial cell, a somatic cell extracted from urine, a gastric mucosal cell, a goblet cell, a gastrin cell/G cell, a B cell, a pericyte cell, an astrocyte, a blood cell and an oligodendrocyte progenitor cell.

20. The method of claim 19,
wherein the somatic cell is derived from a mammal selected from a human, a dog, a cat, a horse, a sheep, a rabbit, a pig, a mice, and a camel.

21. The method of claim 18,
wherein the method further comprises:
culturing the somatic cell introduced with a composition in a culture medium for neuronal differentiation.

22. The method of claim 18,
wherein the introduction is performed by methods selected from electroporation, gene gun, sonoporation, magnetofection, microinjection, transient cell compression or squeezing, cationic liposomal method, lithium acetate-DMSO, lipid-mediated transfection, phosphoric acid Calcium precipitation, lipofection, polyethyleneimine (PEI)-mediated transfection and DEAE-dextran-mediated transfection method.

23. The method of claim 18,
wherein via the introduction, the somatic cell is converted into iMN (induced motor neuron) within 1 to 7 weeks.

24. A pharmaceutical composition for preventing or treating a nervous system disease comprising a Gsta4(Glutathione S-Transferase Alpha 4) protein for a direct cell-conversion of a somatic cell into an iMN (induced motor neuron).

25. The pharmaceutical composition of claim 24,
wherein the pharmaceutical composition further comprises at least one protein selected from Ascl1, Brn2, Myt1L, Hb9, Isl1, Lhx3, Ngn2 and NeuroD1.

26. A pharmaceutical composition for preventing or treating a nervous system disease comprising a somatic cell introduced with a nucleic acid encoding Gsta4(Glutathione S-Transferase Alpha 4) protein for a direct cell-conversion of a somatic cell into an iMN (induced motor neuron).

27. The pharmaceutical composition of claim 26,
wherein the pharmaceutical composition further comprises a nucleic acid encoding at least one protein selected from Ascl1, Brn2, Myt1L, Hb9, Isl1, Lhx3, Ngn2 and NeuroD1.

28. A pharmaceutical composition for preventing or treating a nervous system disease comprising a motor neuron overexpressing direct cell-conversion factors for a direct cell-conversion of a somatic cell into an iMN(induced motor neuron),
wherein the motor neuron overexpresses one or more direct cell-conversion factor selected from Gsta4, Ascl1 (Achaete-scute homolog 1), Brn2 (POU Class 3 Homeobox 2), MytlL (Myelin Transcription Factor 1 Like), Hb9 (Homeobox HB9), ISL1 (ISL LIM homeobox 1), Lhx3 (LIM homeobox 3), Ngn2 (neurogenin 2) and NeuroD1 (neuronal differentiation 1).

29. The pharmaceutical composition of any one of claims 24 to 28,
wherein the pharmaceutical composition further comprises pharmaceutically acceptable a carrier, an excipient, a diluent and a preservative.

30. The pharmaceutical composition of any one of claims 24 to 28,
wherein the nervous system disease is one or more selected from Spinal cord injury, Parkinson's disease, stroke, Huntington's disease, Lou Gehrig's disease, Ataxia telangiectasia, amyotrophic lateral sclerosis, motor neuron damage, traumatic peripheral nerve damage, ischemic brain injury, neonatal hypoxic ischemia Brain damage, cerebral palsy, peripheral palsy, central paralysis, quadriplegia, biparesis, epilepsy, neuronal development disorder, neuralgia, intractable epilepsy, Alzheimer's disease, congenital metabolic nervous system disease, traumatic brain injury, motor nerve cell damage or a disease caused by the motor nerve cell damage.

31. The pharmaceutical composition of any one of claims 24 to 28,
wherein the pharmaceutical composition is in the form of at least one selected from a solid form, a liquid form, a capsule form, and a semi-solid form.

32. A method for preventing or treating a nervous system disease,
the method comprising:
administering i) or ii) below to a subject:
i) a Gsta4(Glutathione S-Transferase Alpha 4) protein; or
ii) a Gsta4(Glutathione S-Transferase Alpha 4) protein; and at least one protein selected from Ascl1, Brn2, Myt1L, Hb9, Isl1, Lhx3, Ngn2 and NeuroD1;
wherein the method does not generate iPSCs (induced pluripotent stem cells).
wherein the administration induces that a somatic cell is directly converted into an iMN (induced motor neuron) without generating iPSCs (induced pluripotent stem cells).

33. A method for preventing or treating a nervous system disease,
the method comprising:
administering a somatic cell introduced with a Gsta4(Glutathione S-Transferase Alpha 4) protein or a nucleic acid encoding the Gsta4 protein to a subject;
wherein the administration induces that a somatic cell is directly converted into an iMN(induced motor neuron) without generating iPSCs(induced pluripotent stem cells).

34. A method for preventing or treating a nervous system disease,
the method comprising:
administering to a subject an iMN (induced motor neuron) induced by introducing a composition comprising a Gsta4(Glutathione S-Transferase Alpha 4) protein or a nucleic acid encoding the Gsta4 protein into a somatic cell;
wherein the administration induces that a somatic cell is directly converted into an iMN (induced motor neuron) without generating iPSCs (induced pluripotent stem cells).

35. The method of any one of claims 33 or 34,
wherein the composition further comprises at least one protein selected from Ascl1, Brn2, Myt1L, Hb9, Isl1, Lhx3, Ngn2 and NeuroD1; or a nucleic acid encoding the same.

36. The method of claim 35,
wherein the composition further comprises a protein of Hb9 and Lhx3; or a nucleic acid encoding the same.

37. The method of any one of claims 32 to 34,
wherein the subject is mammal,
the mammal is selected from a human, a mice, a dog and a cat.

38. The method of any one of claims 32 to 34,
wherein the nervous system disease is one or more selected from spinal cord injury, Parkinson's disease, stroke, amyotrophic spinal lateral sclerosis, motor nerve damage, traumatic peripheral nerve damage, ischemic brain damage, neonatal hypoxic-ischemic brain damage, cerebral palsy, epilepsy, intractable epilepsy, Alzheimer's disease, congenital metabolic nervous system disease, traumatic brain injury, motor nerve cell damage or a disease caused by the motor nerve cell damage.

39. The method of any one of claims 32 to 34,
wherein the administration site is selected from muscle, intradermal, subcutaneous, intravenous, abdominal, arterial, mucosal, spinal, bone marrow, intrathecal and transdermal.

40. wherein the administration is administered at a dose of 1uL/kg to 20uL/kg at a time.
